# EUROPEAN PATENT SPECIFICATION

(11) **EP 2 019 686 B1**
(45) Date of publication and mention of the grant of the patent: **11.07.2012**
(21) Application number: 07773929.0
(22) Date of filing: 29.03.2007
(51) Int. Cl.: A61K 39/21

(54) **COMBINED MUCOSAL AND PARENTERAL IMMUNIZATION AGAINST HIV**
KOMBINIERTE MUKOSALE UND PARENTERALE IMMUNISIERUNG GEGEN HIV
IMMUNISATION MUCOSALE ET PARENTÉRALE COMBINÉE CONTRE LE VIH

(30) Priority: 31.03.2006 US 788563 P; 26.08.2006 US 840178 P
(43) Date of publication of application: 04.02.2009
(60) Divisional application: 11158008.0
(73) Proprietor: Novartis AG, 4056 Basel (CH)
(72) Inventor: RAPPUOLI, Rino, Emeryville, CA 94662-8097 (US); LEWIS, David, J.M., Emeryville, CA 94662-8097 (US); BARNETT, Susan, W., Emeryville, CA 94662-8097 (US)
(74) Representative: Marshall, Cameron John
(86) International application number: PCT/US2007/007583
(87) International publication number: WO 2007/126825

(56) References cited:
- WO-A-02/080648
- WO-A-03/059385
- US-B1- 6 511 845
- VAJDY MICHAEL ET AL: "Mucosal and systemic anti-HIV responses in rhesus macaques following combinations of intranasal and parenteral immunizations" AIDS RESEARCH AND HUMAN RETROVIRUSES, MARY ANN LIEBERT, US, vol. 20, no. 11, November 2004 (2004-11), pages 1269-1281, XP002422502 ISSN: 0889-2229
- SRIVASTAVA INDRESH K ET AL: "Purification, characterization, and immunogenicity of a soluble trimeric envelope protein containing a partial deletion of the V2 loop derived from SF162, an R5-tropic human immunodeficiency virus type 1 isolate" JOURNAL OF VIROLOGY, THE AMERICAN SOCIETY FOR MICROBIOLOGY, US, vol. 77, no. 20, October 2003 (2003-10), pages 11244-11259, XP002332880 ISSN: 0022-538X
- MCCLUSKIE MICHAEL J ET AL: "Parenteral and mucosal prime-boost immunization strategies in mice with hepatitis B surface antigen and CpG DNA" FEMS IMMUNOLOGY AND MEDICAL MICROBIOLOGY, ELSEVIER SCIENCE B.V., AMSTERDAM, NL, vol. 32, no. 3, 18 February 2002 (2002-02-18), pages 179-185, XP002288583 ISSN: 0928-8244
- U.S. N.I.H.: 'Safety Study Of Nasal HIV Vaccine Adjuvanted With LTK63', [Online] 14 April 2008, U.S. N.I.H. Retrieved from the Internet: <URL:http://clinicaltrials.gov/ct2/show/NCT 00369031> [retrieved on 2011-09-13]
- LEWIS D ET AL: "Transient Facial Nerve Paralysis (Bell's Palsy) following Intranasal Delivery of a Genetically Detoxified Mutant of Escherichia coli Heat Labile Toxin", PLOS ONE, vol. 4, no. 9, September 2009 (2009-09), ISSN: 1932-6203, DOI: DOI:10.1371/JOURNAL.PONE.0006999

## Description

### TECHNICAL FIELD

This invention is in the field of human immunodeficiency virus (HIV) and, in particular, compositions and methods for use in HIV-1 immunization.

### BACKGROUND OF THE INVENTION

Vaccines for immunizing patients against HIV infection have been under development for two decades, but with limited success. Many approaches to immunization have focused on the HIV envelope glycoprotein, although there has also been interest in other antigens such as tat or gag.

Reference 1 discloses compositions comprising a HIV antigen, such as an envelope antigen, and a mucosal adjuvant, such as a detoxified mutant of *E.coli* heat labile toxin (LT). The compositions were administered to rhesus macaques intranasally and an antibody response was obtained.

Reference 2 discloses HIV immunization regimens involving parenteral priming and mucosal boosting. Reference 157 discusses HIV immunization regimens involving parenteral and/or mucosal priming and parenteral boosting.

It is an object to provide further and improved ways of mucosally immunizing against HIV.

### SUMMARY OF THE INVENTION

The invention provides an HIV-1 envelope protein (Env) antigen, for use in a therapeutic or prophylactic method for raising serum and mucosal antibody responses in a patient, characterised in that the method comprises: (i) administering a series of priming doses of the HIV-1 envelope protein (Env) antigen to the patient via a mucosal route, wherein the series consists of three doses; and then (ii) administering two boosting doses of an HIV-1 envelope protein (Env) antigen to the patient via the intramuscular route, wherein each immunization uses the same HIV-1 envelope protein (Env) antigen, wherein the first intramuscular dose is administered at least 2 weeks after the final dose in the mucosal series, and wherein administering said boosting doses increases both the serum and mucosal antibody responses induced by said priming doses.

The invention also provides the use of an HIV-1 envelope protein (Env) antigen in the manufacture of a medicament for raising serum and mucosal antibody responses in a patient by a method comprising: (i) administering a series of priming doses of the HIV-1 envelope protein (Env) antigen to the patient via a mucosal route, wherein the series consists of three doses; and then (ii) administering two boosting doses of an HIV-1 envelope protein (Env) antigen to the patient via the intramuscular route, wherein each immunization uses the same HIV-1 envelope protein (Env) antigen, wherein the first intramuscular dose is administered at least 2 weeks after the final dose in the mucosal series, and wherein administering said boosting doses increases both the serum and mucosal antibody responses induced by said priming doses.

The invention also provides an HIV-1 envelope protein (Env) antigen, for use in a therapeutic or prophylactic method for raising serum and mucosal antibody responses in a patient, characterised in that the method comprises: administering two boosting doses of the HIV-1 envelope protein (Env) antigen to the patient via the intramuscular route, wherein the patient has previously received a series of priming doses of an HIV-1 envelope protein (Env) antigen via a mucosal route, wherein the mucosal series consists of three doses, wherein each immunization uses the same HIV-1 envelope protein (Env) antigen, wherein the first intramuscular dose is administered at least 2 weeks after the final dose in the mucosal series, and wherein administering said boosting doses increases both the serum and mucosal antibody responses induced by said priming doses.

The invention also provides the use of an HIV-1 envelope protein (Env) antigen in the manufacture of a medicament for raising serum and mucosal antibody responses in a patient who has previously received a series of priming doses of an HIV-1 envelope protein (Env) antigen via a mucosal route, wherein the mucosal series consists of three doses, wherein the medicament is for administration to the patient via the intramuscular route, wherein two intramuscular boosting doses are administered to said patient, wherein the first intramuscular dose is administered at least 2 weeks after the final dose in the mucosal series, wherein each immunization uses the same HIV-1 envelope protein (Env) antigen, and wherein administering said boosting doses increases both the serum and mucosal antibody responses induced by said priming doses.

The present disclosure relates to methods for raising an immune response in a patient. In one aspect, the method for raising an immune response in a patient comprises: (i) administering a HIV-1 envelope antigen to the patient via a mucosal route; and then (ii) administering a HIV-1 envelope antigen to the patient via a parenteral route. In another aspect, the method for raising an immune response in a patient comprises administering a HIV-1 envelope antigen to the patient via a parenteral route, wherein the patient has previously received a HIV-1 envelope antigen via a mucosal route.

The present disclosure also relates to uses of an HIV-1 envelope antigen in the manufacture of a medicament for administration to a patient by a method comprising: (i) administering the HIV-1 envelope antigen to the patient via a mucosal route; and then (ii) administering a HIV-1 envelope antigen to the patient via a parenteral route.

The disclosure further relates to uses of an HIV-1 envelope antigen in the manufacture of a medicament for administration to a patient who has previously received a HIV-1 envelope antigen via a mucosal route, wherein the medicament is for administration to the patient via a parenteral route.

The present disclosure also relates to kits comprising (i) an HIV-1 envelope antigen for administration to a patient via a mucosal route; and (ii) an HIV-1 envelope antigen for administration to a patient via a parenteral route.

The disclosure further relates to an HIV-1 envelope antigen in combination with a mucosal adjuvant, and a HIV-1 envelope antigen in combination with a parenteral adjuvant, for simultaneous separate or sequential administration.

The present disclosure also relates to immunogenic compositions for intranasal administration. In one aspect, the immunogenic composition for intranasal administration comprises an HIV-1 envelope antigen in combination with a detoxified mutant of *E.coli* heat labile toxin ('LT'), wherein a unit dose of the composition includes between 10µg and 1mg of Env antigen and between 10µg and 50µg of LT. In other aspects, the immunogenic composition for intranasal administration comprises an HIV-1 envelope antigen in combination with a detoxified mutant of *E.coli* heat labile toxin ('LT'), wherein the weight ratio of Env antigen to LT is between 2:1 and 4:1.

The present disclosure further relates to immunogenic compositions for intramuscular administration, comprising an HIV-1 envelope antigen in combination with an oil-in-water emulsion adjuvant, wherein a unit dose of the composition includes between 10µg and 1mg of envelope antigen.

In some embodiments, the HIV-1 envelope antigens of the uses of the invention are adjuvanted. In other embodiments, mucosal immunizations are via the intranasal route. Parenteral immunizations are via the intramuscular route.

In particular embodiments, mucosal immunizations include a detoxified mutant of *E.coli* heat labile toxin as adjuvant. In other embodiments, parenteral immunizations include an oil-in-water emulsion as adjuvant.

Three mucosal immunizations are administered in the uses of the invention. In some embodiments, mucosal immunization is intranasal. In certain embodiments, the mucosal immunization has three intranasal doses of a composition with between 10µg and 50µg of LT-K63 adjuvant per dose. More than one parenteral immunization is administered in the uses of the invention. Parenteral immunization is intramuscular. In certain embodiments, the parenteral immunization has two intramuscular doses of a composition with a squalene-in-water emulsion.

Each immunization uses the same HIV-1 envelope antigen. In other aspects of the disclosure, each immunization uses a different HIV-1 envelope antigen. In a particular embodiment, the envelope antigen is o-gp140ΔV2_{SF162}.

The uses of the invention can be used (employed) in cases where the patient is not infected with HIV-1. The uses of the invention can also be employed (used) where the patient is infected with HIV-1. In particular embodiments, the patient is a human aged between 12 and 55 years old.

### BRIEF DESCRIPTION OF THE DRAWINGS

FIG. 1 shows IgG responses in mice after three intranasal doses.
FIG. 2 shows the same responses after the mice received two further intramuscular doses.
FIG. 3 shows IgA responses for the same mice as in Figure 2.

### DETAILED DESCRIPTION OF THE INVENTION

Reference 2 discloses HIV immunization regimens involving parenteral priming and mucosal boosting. In contrast, in the present invention, a HIV-1 envelope protein antigen is for a use as recited in the claims, entailing a regimen comprising administration mucosally in priming immunizations, and then parenterally in boosting immunizations. The initial mucosal immunization primes both the systemic immune compartment and, via the common mucosal immune system, seed cells in diverse and distant mucosal sites such as the rectum, vagina and cervix. Both T cell and antibody immunity can be elicited at these mucosal sites. The primary antibody and CD4+ T cell responses induced by the mucosal immunizations can be amplified by the parenteral booster immunizations. Compared to the mucosal administration used in reference 1, the data herein show that addition of parenteral boosting increases both the serum and mucosal antibody responses.

Thus disclosed herein is a method for raising an immune response in a patient, comprising: (i) administering a HIV-1 envelope antigen to the patient via a mucosal route; and then (ii) administering a HIV-1 envelope antigen to the patient via a parenteral route.

Disclosed herein is a method for raising an immune response in a patient, comprising: administering a HIV-1 envelope antigen to the patient via a parenteral route, wherein the patient has previously received a HIV-1 envelope antigen via a mucosal route.

Disclosed herein is the use of a HIV-1 envelope antigen in the manufacture of a medicament for administration to a patient by a method comprising: (i) administering the HIV-1 envelope antigen to the patient via a mucosal route; and then (ii) administering a HIV-1 envelope antigen to the patient via a parenteral route.

Disclosed herein is the use of a HIV-1 envelope antigen in the manufacture of a medicament for administration to a patient who has previously received a HIV-1 envelope antigen via a mucosal route, wherein the medicament is for administration to the patient via a parenteral route.

Disclosed herein is a kit comprising (i) a HIV-1 envelope antigen for administration to a patient via a mucosal route; and (ii) a HIV-1 envelope antigen for administration to a patient via a parenteral route. The kit may be combined (e.g. in the same box) with a leaflet including details of the composition e.g. instructions for administration, details of the antigens within the composition, *etc.* The instructions may also contain warnings *e.g.* to keep a solution of adrenaline readily available in case of anaphylactic reaction following vaccination, *etc.*

The antigens will typically be adjuvanted.

Thus disclosed herein is (i) a HIV-1 envelope antigen in combination with a mucosal adjuvant; and (ii) a HIV-1 envelope antigen in combination with a parenteral adjuvant, for simultaneous separate or sequential administration.

Preferred mucosal immunizations are via the intranasal route using a detoxified mutant of *E.coli* heat labile toxin as the adjuvant. Preferred immunizations via the intramuscular route use an adjuvant capable of inducing (eliciting) a Th1-type immune response in a subject. In a particular embodiment, the adjuvant is an oil-in-water emulsion adjuvant.

Also disclosed herein is an immunogenic composition for intranasal administration, comprising a HIV envelope antigen in combination with a detoxified mutant of *E.coli* heat-labile toxin ('LT'). A unit dose of the composition may include between 10µg and 1mg of envelope antigen e.g. about 100µg. A unit dose of the composition may include between 10µg and 50µg of LT e.g. about 30µg. A unit dose for intranasal administration typically has a volume of about 300µl. This composition can be used in the uses of the invention.

Also disclosed herein is an immunogenic composition for intranasal administration, comprising a HIV-1 envelope antigen in combination with a detoxified mutant of *E.coli* heat-labile toxin ('LT'), wherein the weight ratio of envelope antigen to LT is between 2:1 and 4:1 *e.g*. about 10:3. This composition can be used in the uses of the invention.

Also disclosed herein is an immunogenic composition for intramuscular administration, comprising a HIV-1 envelope antigen in combination with an adjuvant capable of inducing (eliciting) a Th1-type immune response in a subject. In a particular aspect, the adjuvant is an oil-in-water emulsion adjuvant. A unit dose of the composition may include between 10µg and 1mg of envelope antigen *e.g.* about 100µg. This composition can be used in the uses of the invention.

### The HIV-1 envelope antigen(s)

Where more than one mucosal immunization is administered, the HIV envelope antigen(s) in each immunization may be the same as or different from each other. Similarly, where more than one parenteral immunization is administered, the HIV envelope antigen(s) in each immunization may be the same as or different from each other. Moreover, the HIV envelope antigen(s) that is/are mucosally administered may be the same as or different from the HIV envelope antigen(s) that is/are parenterally administered. In the invention the mucosal immunizations and the parenteral administrations will use the same HIV envelope antigen.

Where different HIV envelope antigens are used, the antigens may be from the same group *e.g.* both are from group M, group N or group O. Within group M, the envelope antigens may be from the same subtype (or clade) *e.g*. from subtype A, B, C, D, F, G, H, J or K. It is also possible to use envelope antigens from a CRF (circulating recombinant form) subtype, such as a A/B or A/E CRF. Where a subtype includes sub-subtypes then the envelope antigens may be from the same sub-subtype. In other aspects, envelope antigens are from different groups, subtypes and/or sub-subtypes. HIV-1 nomenclature is discussed in more detail in reference 3.

The HIV-1 envelope protein (Env) is initially expressed as a long precursor protein ('gp160') that is subsequently cleaved to give an exterior membrane glycoprotein ('gp120') and a transmembrane glycoprotein ('gp41'). The invention can use various forms of Env protein. For example, the invention may use a full-length gp160 polypeptide, a gp120 polypeptide, a gp160 or gp120 polypeptide with one or more deletions, a fusion protein including a gp 120 or gp160 polypeptide, *etc*. Rather than being a full-length gp160 precursor, however, the invention will typically use a shortened protein.

The amino acid sequence of the full-length HIV-1 Env precursor from the REFSEQ database (GI:9629363) is a 856mer shown below (SEQ ID NO: 1 herein):

MRVKEKYQHLWRWGWRWGTMLLGMLMICSATEKLWVTVYYGVPVWKEATTTLFCASDAKAYDTEVHNVWATHA
CVPTDPNPQEVVLVNVTENFNMWKNDMVEQMHEDIISLWDQSLKPCVKLTPLCVSLKCTDLKNDTNTNSSSGR
MIMEKGEIKNCSFNISTSIRGKVQKEYAFFYKLDIIPIDNDTTSYKLTSCNTSVITQACPKVSFEPIPIHYCA
PAGFAILKCNNKTFNGTGPCTNVSTVQCTHGIRPVVSTQLLLNGSLAEEEVVIRSVNFTDNAKTIIVQLNTSV
EINCTRPNNNTRKRIRIQRGPGRAFVTIGKIGNMRQAHCNISRAKWNNTLKQIASKLREQFGNNKTIIFKQSS
GGDPEIVTHSFNCGGEFFYCNSTQLFNSTWFNSTWSTEGSNNTEGSDTITLPCRIKQIINMWQKVGKAMYAPP
ISGQIRCSSNITGLLLTRDGGNSNNESEIFRPGGGDMRDNWRSELYKYKVVKIEPLGVAPTKAKRRVVQREKR
AVGIGALFLGFLGAAGSTMGAASMTLTVQARQLLSGIVQQQNNLLRAIEAQQHLLQLTVWGIKQLQARILAVE
RYLKDQQLLGIWGCSGKLICTTAVPWNASWSNKSLEQIWNHTTWMEWDREINNYTSLIHSLIEESQNQQEKNE
QELLELDKWASLWNWFNITNWLWYIKLFIMIVGGLVGLRIVFAVLSIVNRVRQGYSPLSFQTHLPTPRGPDRP
EGIEEEGGERDRDRSIRLVNGSLALIWDDLRSLCLFSYHRLRDLLLIVTRIVELLGRRGWEALKYWWNLLQYW
SQELKNSAVSLLNATAIAVAEGTDRVIEVVQGACRAIRHIPRRIRQGLERILL

The wild-type HIV-1 precursor protein is cleaved to give the surface glycoprotein gp120 (*e.g*. amino acids 29-511 of SEQ ID NO: 1; SEQ ID NO: 2 herein) and the transmembrane domain gp41 (*e.g*. amino acids 512-856 of SEQ ID NO: 1; SEQ ID NO: 3 herein):

MRVKEKYQHLWRWGWRWGTMLLGMLMIC/SATEKLWVTVYYGVPVWKEATTTLFCASDAKAYDTEVHNVWATH
ACVPTDPNPQEVVLVNVTENFNMWKNDMVEQMHEDIISLWDQSLKPCVKLTPLCVSLKCTDLKNDTNTNSSSG
RMIMEKGEIKNCSFNISTSIRGKVQKEYAFFYKLDIIPIDNDTTSYKLTSCNTSVITQACPKVSFEPIPIHYC
APAGFAILKCNNKTFNGTGPCTNVSTVQCTHGIRPVVSTQLLLNGSLAEEEVVIRSVNFTDNAKTIIVQLNTS
VEINCTRPNNNTRKRIRIQRGPGRAFVTIGKIGNMRQAHCNISRAKWNNTLKQIASKLREQFGNNKTIIFKQS
SGGDPEIVTHSFNCGGEFFYCNSTQLFNSTWFNSTWSTEGSNNTEGSDTITLPCRIKQIINMWQKVGKAMYAP
PISGQIRCSSNITGLLLTRDGGNSNNESEIFRPGGGDMRDNWRSELYKYKVVKIEPLGVAPTKAKRRVVQREK
R/AVGIGALFLGFLGAAGSTMGAASMTLTVQARQLLSGIVQQQNNLLRAIEAQQHLLQLTVWGIKQLQARILA
VERYLKDQQLLGIWGCSGKLICTTAVPWNASWSNKSLEQIWNHTTWMEWDREINNYTSLIHSLIEESQNQQEK
NEQELLELDKWASLWNWFNITNWLWYIKLFIMIVGGLVGLRIVFAVLSIVNRVRQGYSPLSFQTHLPTPRGPD
RPEGIEEEGGERDRDRSIRLVNGSLALIWDDLRSLCLFSYHRLRDLLLIVTRIVELLGRRGWEALKYWWNLLQ
YWSQELKNSAVSLLNATAIAVAEGTDRVIEVVQGACRAIRHIPRRIRQGLERILL

The hypervariable regions within the gp120 region are located as follows, numbered according to SEQ ID NO: 1: V1 = 131-157; V2 = 157-196; V3 = 296-331; V4 = 385-418; and V5 = 461-471. Within the overall C1-V1-V2-C2-V3-C3-V4-C4-V5-C5 arrangement of gp120, therefore, the subdomains are as follows (numbered according to SEQ ID NO: 2): 1-102; 103-129; 129-168; 169-267; 268-303; 304-356; 357-390; 391-432; 433-443; and 444-483. Residues that have been identified as important for CD4 binding include (numbered according to SEQ ID NO: 1) Asp-368, Glu-370, Trp-427, Val-430 and Pro-438, and the immunodominant region is residues 588-607. These features can be identified in other HIV-1 Env sequences by performing a suitable sequence alignment. Pre-aligned sequences from numerous strains, annotated with these features, can also be found in the *Los Alamos HIV Sequence Compendia* [4].

Other Env sequences that can be used include those disclosed in references 5 to 9

As mentioned above, the invention will typically use a shortened Env polypeptide. The shortening will involve the removal of one of more amino acids from the full-length sequence *e.g*. truncation at the C-terminus and/or N-terminus, deletion of internal residues, removal of subdomains [10], and combinations of these approaches.

For instance, it is known to make a soluble form of the Env precursor by removing its transmembrane domain and cytoplasmic tail. This polypeptide, which includes the gp120 sequence and the ectodomain of gp41, is known as 'gp140' [11], and has been reported to be a better immunogen than gp120 [12]. Thus the precursor is truncated at its C-terminus *e.g*. after Lys-665 of SEQ ID NO:1, giving a mature gp140 sequence of a 637mer (SEQ ID NO:4 herein) having amino acids Ser-29 to Lys-665 of SEQ ID NO: 1. Thus the Env polypeptide may include a portion of gp41 but not include its transmembrane domain.

It is also known to make deletions within the V2 loop of the Env precursor, to give 'ΔV2' mutants. For instance, one or more amino acids within the 40-mer V2 loop can be deleted (*e.g*. at least 2, 3, 4, 5, 6, 7, 8, 9, 10, 12, 14, 16, 18, 20, 22, 24, 26, 28, 30, 32, 34, 36, 38 or more amino acids). Deletions within the V2 loop have been reported to improve immunogenicity of Env polypeptides [13,14]. Env polypeptides with deletions and/or substitutions in the V2 loop are preferred with the present invention, as these have been found to be particularly useful in forming Env/Tat complexes. In particular, Env/Tat complexes are not seen with monomeric gp120 unless its V2 loop is mutated. Amino acids deleted from the V2 loop may be substituted with other amino acids *e.g.* it is known to replace the central portion of the V2 loop with a Gly-Ala-Gly tripeptide. For example, a AV2 mutant may have the following sequence (SEQ ID NO: 5): 
SATEKLWVTVYYGVPVWKEATTTLFCASDAKAYDTEVHNVWATHACVPTDPNPQEVVLVNVTENFNMWKNDMV
EQMHEDIISLWDQSLKPCVKLTPLCVSLKCTDLKNDTNTNSSSGRMIMEKGEIKNCXCNTSVITQACPKVSFE
PIPIHYCAPAGFAILKCNNKTFNGTGPCTNVSTVQCTHGIRPVVSTQLLLNGSLAEEEVVIRSVNFTDNAKTI
IVQLNTSVEINCTRPNNNTRKRIRIQRGPGRAFVTIGKIGNMRQAHCNISRAKWNNTLKQIASKLREQFGNNK
TIIFKQSSGGDPEIVTHSFNCGGEFFYCNSTQLFNSTWFNSTWSTEGSNNTEGSDTITLPCRIKQIINMWQKV
GKAMYAPPISGQIRCSSNITGLLLTRDGGNSNNESEIFRPGGGDMRDNWRSELYKYKVVKIEPLGVAPTKAKR
RVVQREKR
 where the 'X' at position 130 represents a mutant V2 loop e.g. with between 4 and 15 amino acids.

A particularly preferred Env polypeptide for use with the invention is a gp140 protein with a ΔV2 mutation from HIV-1 strain SF162. In its mature form, after cleavage of a signal sequence and secretion (see Figure 24 of reference 5), this polypeptide has the following amino acid sequence (SEQ ID NO: 6; gp140ΔV2_{SF162}'):

SAVEKLWVTVYYGVPVWKEATTTLFCASDAKAYDTEVHNVWATHACVPTDPNPQEIVLENVTENFNMWKNNMV
EQMHEDIISLWDQSLKPCVKLTPLCVTLHCTNLKNATNTKSSNWKEMDRGEIKNCSFKVGAGKLINCNTSVIT
QACPKVSFEPIPIHYCAPAGFAILKCNDKKFNGSGPCTNVSTVQCTHGIRPVVSTQLLLNGSLAEEGVVIRSE
NFTDNAKTIIVQLKESVEINCTRPNNNTRKSITIGPGRAFYATGDIIGDIRQAHCNISGEKWNNTLKQIVTKL
QAQFGNKTIVFKQSSGGDPEIVMHSFNCGGEFFYCNSTQLFNSTWNNTIGPNNTNGTITLPCRIKQIINRWQE
VGKAMYAPPIRGQIRCSSNITGLLLTRDGGKEISNTTEIFRPGGGDMRDNWRSELYKYKVVKIEPLGVAPTKA
ISSVVQSEKSAVTLGAMFLGFLGAAGSTMGARSLTLTVQARQLLSGIVQQQNNLLRAIEAQQHLLQLTVWGIK
QLQARVLAVERYLKDQQLLGIWGCSGKLICTTAVPWNASWSNKSLDQIWNNMTWMEWEREIDNYTNLIYTLIE
ESQNQQEKNEQELLELDKWASLWNWFDISKWLWYI

As the HIV genome is in a state of constant flux, and contains several domains that exhibit relatively high degrees of variability between isolates, the invention is not limited to the use of Env polypeptides having the exact sequence of a known HIV polypeptide. Thus the Env polypeptide used according to the invention may be selected from:
(i) a polypeptide comprising an amino acid sequence selected from SEQ ID NOs: 1, 2, 4, 5 and 6;
(ii) a polypeptide comprising an amino acid sequence that has sequence identity to an amino acid sequence selected from SEQ ID NOs: 1, 2, 4, 5 and 6;
(iii) a polypeptide comprising an amino acid sequence that, compared to an amino acid sequence selected from SEQ ID NOs: 1, 2, 4, 5 and 6, has one or more (*e.g*. 1, 2, 3, 4, 5, 6, 7, 8, 9, 10, *etc*.) substitutions and/or deletions and/or insertions;
(iv) a polypeptide comprising an amino acid sequence comprising a fragment of at least *n* consecutive amino acids from an amino acid sequence selected from SEQ ID NOs: 1, 2, 4, 5 and 6, where *n* is 7 or more; or
(v) a polypeptide comprising a sequence of *p* amino acids that, when aligned with an amino acid sequence selected from SEQ ID NOs: 1, 2, 4, 5 and 6 using a pairwise alignment algorithm, has at least *x·y* identical aligned monomers in each window of *x* amino acids moving from N-terminus to C-terminus, where: *p*>*x*; there are *p*-*x*+*1* windows; *x* is selected from 20, 25, 30, 35, 40, 45, 50, 60, 70, 80, 90, 100, 150, 200, 250, 300, 350, 400, 450, 500, 550, 600, 650, 700, 750, 800 or 850; *y* is selected from 0.50, 0.60, 0.70, 0.75, 0.80, 0.85, 0.90, 0.91, 0.92, 0.93, 0.94, 0.95, 0.96, 0.97, 0.98, or 0.99; and, if *x·y* is not an integer, it is rounded up to the nearest integer.

These polypeptides include homologs, orthologs, allelic variants and mutants of SEQ ID NOs 1, 2, 4, 5 and 6. For instance, it is known to mutate natural Env sequences to improve resistance to proteases. The polypeptides also include fusion polypeptides, in which the Env sequence is fused to non-Env sequence. For instance, it is known to fuse Env sequences without the native leader peptide to leader peptides from non-Env proteins *e.g*. from tissue plasminogen activator.

Within category (ii), the degree of sequence identity may be greater than 50% (*e.g*. 60%, 70%, 75%, 80%, 85%, 90%, 91%, 92%, 93%, 94%, 95%, 96%, 97%, 98%, 99% or more). Identity between polypeptides is preferably determined by the Smith-Waterman homology search algorithm as implemented in the MPSRCH program (Oxford Molecular), using an affine gap search with parameters *gap open penalty*=*12* and *gap extension penalty*= *1*.

Within category (iii), each substitution involves a single amino acid, each deletion preferably involves a single amino acid, and each insertion preferably involves a single amino acid. These changes may arise deliberately (*e.g.* by site-directed mutagenesis) or naturally (*e.g*. through virus evolution or through spontaneous mutation). The polypeptides in category (iii) may have one or more (*e.g.* 1, 2, 3, 4, 5, 6, 7, 8, 9, 10, *etc.*) single amino acid substitutions relative to SEQ ID NO: 1, 2, 4, 5 or 6. These polypeptides may have one or more (*e.g*. 1, 2, 3, 4, 5, 6, 7, 8, 9, 10, *etc*.) single amino acid deletions relative to SEQ ID NO: 1, 2, 4, 5 or 6. These polypeptide s may have one or more (*e.g*. 1, 2, 3, 4, 5, 6, 7, 8, 9, 10, *etc*.) single amino acid insertion relative to SEQ ID NO: 1, 2, 4, 5 or 6. The substitutions, insertions and/or deletions may be at separate locations or may be contiguous. Substitutions may be conservative *i.e*. replacements of one amino acid with another which has a related side chain. Genetically-encoded amino acids are generally divided into four families: (1) acidic *i.e.* aspartate, glutamate; (2) basic *i.e* lysine, arginine, histidine; (3) non-polar *i.e*. alanine, valine, leucine, isoleucine, proline, phenylalanine, methionine, tryptophan; and (4) uncharged polar *i.e*. glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. Various substitutions have been described for use with Env polypeptides *e.g*. it is known to inactivate the cleavage site between gp120 and gp41 (*e.g*. by a Lys-Ser substitution) in order to provide a polypeptide that remains in full-length form, or to remove the 'clipping' site in the V3 loop [15], or to delete or substitute glycosylation sites, particularly N-glycosylation sites (*i.e*. asparagine residues). Within category (iv), the value of *n* may be greater than 7 *e.g.* 8, 10, 12, 14, 16, 18, 20, 22, 24, 26, 28, 30, 35, 40, 45, 50, 60, 70, 80, 90, 100, 150, 200, 250, 300, 350, 400, 450, 500, 550, 600, 650, 700, 750, 800, 850 or more. The fragment may comprise at least one T-cell and/or B-cell epitope of the sequence. T- and B-cell epitopes can be identified empirically (*e.g*. using PEPSCAN [16,17] or similar methods), or they can be predicted (*e.g*. using the Jameson-Wolf antigenic index [18], matrix-based approaches [19], TEPTTOPE [20], neural networks [21], OptiMer & EpiMer [22,23], ADEPT [24], Tsites [25], hydrophilicity [26], antigenic index [27] or the methods disclosed in ref. 28, *etc.*). Within category (v), the preferred pairwise alignment algorithm is the Needleman-Wunsch global alignment algorithm [29], using default parameters (*e.g*. with Gap opening pe nalty = 10.0, and with Gap extension penalty = 0.5, using the EBLOSUM62 scoring matrix). This algorithm is conveniently implemented in the *needle* tool in the EMBOSS package [30].

Within this group of Env polypeptides that may be used with the invention, a preferred feature is that the polypeptide should retain the ability of natural Env to bind to CD4.

Env polypeptide is found in oligomeric form on the HIV virion, and preferred Env polypeptides used with the invention can also form oligomers, and in particular trimers. For instance, ΔV2 mutants of gp140 have been shown to form trimers [13] (sometimes referred to as 'o-gp140').

The Env protein may be used in combination with one or more further HIV antigens, such as Gag, Pol, Env, Tat, Rev, Nef, Vpr, Vpu and/or Vif. For instance, reference 1 uses combinations of Env (*e.g.* o-gp140) and Gag (*e.g.* p24). Similarly, reference 31 discloses the use of a combination of Env and Tat polypeptides, and reference 32 uses a complex of Env and Tat proteins for immunization. These and other mixtures can be used with the invention.

### The mucosal immunizadon(s)

The invention involves an initial mucosal immunization with HIV antigen(s). A patient may receive a single mucosal dose, or may receive a series of multiple mucosal doses (*e.g*. 2, 3, 4, 5, 6, 7, 8 or more mucosal doses). The regimen of the invention involves 3 mucosal immunizations.

Where multiple doses are administered, each dose after the first dose will typically be administered at least 2 weeks after the previous one *e.g.* ≥3 weeks apart, ≥4 weeks apart, ≥6 weeks apart, ≥8 weeks apart, ≥12 weeks apart, *etc*. A typical 3-dose schedule may be at 0, 4 and 8 weeks, where 0 is the time of the first dose.

Routes for mucosal administration of compositions include intranasal [33-35], intragastric, rectal, vaginal, peroral [36], pulmonary, intestinal, intradermal, transdermal, buccal, sublingual, *etc*. Where more than one mucosal immunization is given, each of them is preferably via the same route. The preferred route for mucosal administration is the intranasal route, as it offers easy access with relatively simple devices that have already been mass produced. The composition of the disclosure may thus be presented for intranasal administration, such as by nasal spray, nasal drops, gel or powder (*e.g.* refs 37 & 38). Mucosal administration may also involve presenting the composition in the form of tablets or capsules (optionally enteric-coated), liquid, transgenic plant material, drops, inhalers, aerosol, suppositories, pessaries, *etc*. (see also ref. 39, and Chapter 17 of ref. 57).

Antigens given by a mucosal route will typically be administered in combination with (*e.g*. in admixture with) a suitable adjuvant. Adjuvants that are active via mucosal routes include, but are not limited to:
- Bacterial ADP-ribosylating toxins (*e.g*. the *E.coli* heat labile enterotoxin "LT", cholera toxin "CT", or pertussis toxin "PT") and detoxified derivatives thereof, such as the mutant toxins known as LT-K63 and LT-R72 [40]. Mutagenesis can be used to remove the toxic enzymatic activity of these toxins without removing their adjuvant activity. The use of detoxified ADP-ribosylating toxins as mucosal adjuvants is described in reference 41.
- Microparticles (*i.e*. a particle of ~100nm to ~150pm in diameter, more preferably ~200nm to ~30µm in diameter, and most preferably ~500nm to ~10µm in diameter) formed from materials that are biodegradable and non-toxic (*e.g*. a poly(a-hydroxy acid), a polyhydroxybutyric acid, a polyorthoester, a polyanhydride, a polycaprolactone *etc*., such as poly(lactide-co-glycolide) *etc*.) optionally treated to have a negatively-charged surface (*e.g*. with SDS) or a positively-charged surface (*e.g*. with a cationic detergent, such as CTAB).

- A polyoxyethylene ether or a polyoxyethylene ester [42]. Such adjuvant formulations further include polyoxyethylene sorbitan ester surfactants in combination with an octoxynol [43] as well as polyoxyethylene alkyl ethers or ester surfactants in combination with at least one additional non-ionic surfactant such as an octoxynol [44]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.
- Bioadhesives [45] and mucoadhesives, such as esterified hyaluronic acid microspheres [46], chitosan [47] and its derivatives [48], cross-linked derivatives of poly(acrylic acid), polyvinyl alcohol, polyvinyl pyrollidone, polysaccharides and carboxymethylcellulose.
- An immunostimulatory oligonucleotide (*e.g*. a CpG oligonucleotide) and a saponin [49].
- Liposomes (chapters 13 & 14 of ref. 57]. Examples of liposome formulations suitable for use as adjuvants are described in refs. 50-52.
- Aminoalkyl glucosaminide phosphate derivatives, such as RC-529 [53,54].
- A phosphazene, such as poly[di(carboxylatophenoxy)phosphazene] ("PCPP") as described, for example, in references 55 and 56.

Compositions may include two or more of said adjuvants.

Other adjuvants that are active via mucosal routes are also available (*e.g*. see chapter 7 of ref. 57).

A preferred class of mucosal adjuvants is the group of ADP-ribosylating toxins. Within this group, detoxified toxins are preferred. Detoxified mutants of LT and CT are preferred. In one embodiment, LT having a mutation at residue Ala-72 (*e.g*. an Arg substitution [58], to give 'LT-R72') can be used. In another embodiment, LT having a mutation at residue Ser-63 (*e.g*. a Lys substitution [Chapter 5 of ref. 59] to give 'LT-K63', or a Tyr substitution [60]). Numbering of these residues is according to reference 61. The LT-K63 mutant is particularly suitable for use with the invention.

Thus a preferred mucosal immunization used with the invention involves multiple doses (*e.g*. 3) by the intranasal route using LT-K63 adjuvant. A typical intranasal composition will include between 10µg and 50µg of LT adjuvant per dose *e.g.* about 30µg of LT-K63 per dose.

### The parenteral immunization(s)

After an initial mucosal immunization, envelope antigen is administered parenterally. A patient may receive a single parenteral dose, or may receive a series of multiple parenteral doses (*e.g*. 2, 3, 4, 5, 6, 7, 8 or parenteral more doses). The regimen of the invention involves 2 intramuscular immunizations.

The first (or only) parenteral dose will typically be administered at least 2 weeks after the final dose in the mucosal series *e.g.* ≥3 weeks after, ≥4 weeks after, ≥6 weeks after, ≥8 weeks after, ≥12 weeks after, *etc.* The first parenteral dose may be administered, for instance, about 8 weeks after the final (*e.g.* third) mucosal dose.

Where multiple parenteral doses are administered, each dose after the first parenteral dose will typically be administered at least 2 weeks after the previous one *e.g*. ≥3 weeks apart, ≥4 weeks apart, ≥6 weeks apart, ≥8 weeks apart, ≥12 weeks apart, *etc*. A typical 2-dose schedule may be at 0 and 12 weeks, where 0 is the time of the first parenteral dose (*e.g*. at 16 & 28 weeks relative to the first mucosal dose.)

Routes for parenteral administration of compositions include subcutaneous, intravenous, intramuscular, *etc.* Administration via these routes will typically be by injection. Where more than one parenteral immunization is given, each of them is preferably via the same route. The route for parenteral administration according to the invention is by intramuscular injection.

Antigens given by a parenteral route will typically be administered in combination with (e.g. in admixture with) a suitable adjuvant. Particularly suitable adjuvants are capable of inducing (eliciting) a Th1-type immune response in a subject. Such adjuvants are also referred to herein as Th1 adjuvants. The distinction between Th1 and Th2 T helper cells is well known. Th1 and Th2 adjuvants cause an immune response to a co-administered antigen to be biased towards, respectively, a Th1-type or a Th2-type response. Thus Th1 adjuvants result in the production of antigen-specific T cells that release cytokines such as IL-2 and interferon-γ (leading to IgG2a antibodies), whereas Th2 adjuvants result in the production of antigen-specific T cells that release cytokines such as IL-4 (leading to IgG1).

Th1-type responses are naturally linked to bacterial infections, and so adjuvants used with the invention generally include substances that mimic a bacterial substance. Th1 adjuvants may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (*e.g*. imidazoquinolines) and/or TLR9 (*e.g*. CpG oligonucleotides). These agents are useful for activating innate immunity pathways.

Adjuvants that are active for parenterally administered antigens include, but are not limited to:
- An oil-in-water emulsion, as described in more detail below.
- An immunostimulatory oligonucleotide, such as one containing a CpG motif (a dinucleotide sequence containing an unmethylated cytosine linked by a phosphate bond to a guanosine), a TpG motif [62],a double-stranded RNA, an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded. References 63 to 65 disclose possible analog substitutions *e.g*. replacement of guanosine with 2'-deoxy-7-deazaguanosine. The adjuvant effect of CpG oligonucleotides is further discussed in refs. 66-71. A CpG sequence may be directed to TLR9, such as the motif GTCGTT or TTCGTT [72]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 73-75. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 72 & 76-78. A useful CpG adjuvant is CpG7909, also known as ProMune™ (Coley Pharmaceutical Group, Inc.). Immunostimulatory oligonucleotides will typically comprise at least 20 nucleotides. They may comprise fewer than 100 nucleotides.
- 3-O-deacylated monophosphoryl lipid A ('3dMPL', also known as 'MPLT™') [79-82]. 3dMPL has been prepared from a heptoseless mutant of *Salmonella minnesota,* and is chemically similar to lipid A but lacks an acid-labile phosphoryl group and a base-labile acyl group. Preparation of 3dMPL was originally described in reference 83. 3dMPL can take the form of a mixture of related molecules, varying by their acylation (*e.g*. having 3, 4, 5 or 6 acyl chains, which may be of different lengths). The two glucosamine (also known as 2-deoxy-2-amino-glucose) monosaccharides are N-acylated at their 2-position carbons (*i.e*. at position 2 and 2'), and there is also O-acylation at the 3' position. 3dMPL promotes a Th1-type response [84].
- An imidazoquinoline compound, such as Imiquimod ("R-837") [85,86], Resiquimod ("R-848") [87], and their analogs; and salts thereof (*e.g*. the hydrochloride salts). Further details about immunostimulatory imidazoquinolines can be found in references 88 to 92. These compounds shift responses towards Th1.
- A thiosemicarbazone compound, such as those disclosed in reference 93. Methods of formulating, manufacturing, and screening for active compounds are also described in reference 93. The thiosemicarbazones are particularly effective in the stimulation of human peripheral blood mononuclear cells for the production of cytokines, such as TNF-α.
- A tryptanthrin compound, such as those disclosed in reference 94. Methods of formulating, manufacturing, and screening for active compounds are also described in reference 94. The thiosemicarbazones are particularly effective in the stimulation of human peripheral blood mononuclear cells for the production of cytokines, such as TNF-α.
- A mineral-containing composition, including calcium salts and aluminum salts (or mixtures thereof). Calcium salts include calcium phosphate (*e.g*. the "CAP" particles disclosed in ref. 95). Aluminum salts include hydroxides, phosphates, sulfates, *etc.,* with the salts taking any suitable form (*e.g.* gel, crystalline, amorphous, *etc*.). Adsorption to these salts is preferred. The mineral containing compositions may also be formulated as a particle of metal salt [96]. Aluminum salt adjuvants are described in more detail below.
- A nucleoside analog, such as: (a) Isatorabine (ANA-245; 7-thia-8-oxoguanosine): 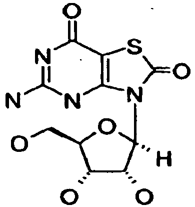 and prodrugs thereof; (b) ANA975; (c) ANA-025-1; (d) ANA380; (e) the compounds disclosed in references 97 to 99; (f) a compound having the formula: 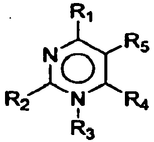
wherein:
R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 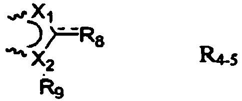 the binding being achieved at the bonds indicated by a 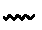
X₁ and X₂ are each independently N, C, O, or S;
R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚR_{c}, or -C(O)-R_{d};
R₉ is H, C₁₋₆ alkyl, substituted C₁₋₆ alkyl, heterocyclyl, substituted heterocyclyl or R₉ₐ, wherein R₉ₐ is: 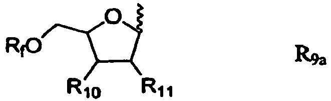 the binding being achieved at the bond indicated by a
R₁₀ and R₁₁ are each independently H, halo, C₁₋₆ alkoxy, substituted C₁₋₆ alkoxy, -NRₐR_{b}, or -OH;
each Rₐ and R_{b} is independently H, C₁₋₆ alkyl, substituted C₁₋₆ alkyl, -C(O)Rₐ, C₆₋₁₀ aryl;
each R_{c} is independently H, phosphate, diphosphate, triphosphate, C₁₋₆ alkyl, or substituted C₁₋₆ alkyl;
each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl, -N(substituted C₁₋₆ alkyl)₂, C₆₋₁₀ aryl, or heterocyclyl;
each R_{c} is independently H, C₁₋₆ alkyl, substituted C₁₋₆ alkyl, C₆₋₁₀ aryl, substituted C₆₋₁₀ aryl, heterocyclyl, or substituted heterocyclyl;
each R_{f} is independently H, C₁₋₆ alkyl, substituted C₁₋₆ alkyl, -C(O)R_{d}, phosphate, diphosphate, or triphosphate;
each n is independently 0, 1, 2, or 3;
each p is independently 0, 1, or 2; or
or (g) a pharmaceutically acceptable salt of any of (a) to (f), a tautomer of any of (a) to (f), or a pharmaceutically acceptable salt of the tautomer.
- Loxoribine (7-allyl-8-oxoguanosine) [100].
- Compounds disclosed in reference 101, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [102,103], Hydrapthalamide compounds, Benzophenone compounds, Isoxazole compounds, Sterol compounds, Quinazilinone compounds, Pyrrole compounds [104], Anthraquinone compounds, Quinoxaline compounds, Triazine compounds, Pyrazalopyrimidine compounds, and Benzazole compounds [105].
- Compounds disclosed in reference 106, including 3,4-di(1H-indol-3-yl)-1H-pyrrole-2,5-diones, staurosporine analogs, derivatized pyridazines, chromen-4-ones, indolinones, quinazolines, and nucleoside analogs.
- An aminoalkyl glucosaminide phosphate derivative, such as RC-529 (see above). These derivatives stimulate Th1 responses [107].
- A phosphazene, as described above.
- Small molecule immunopotentiators (SMIPs) such as:
   N2-methyl-1-(2-methylpropyl)-1H-imidazo[4,5-c]quinoline-2,4-diamine
   N2,N2-dimethyl-1-(2-methylpropyl)-1H-imidazo[4,5-c]quinoline-2,4-diamine
   N2-ethyl-N2-methyl-1-(2-methylpropyl)-1H-imidazo[4,5-c]quinoline-2,4-diamine
   N2-methyl-1-(2-methylpropyl)-N2-propyl-1H-imidazo[4,5-c]quinoline-2,4-diamine
   1-(2-methylpropyl)-N2-propyl-1H-imidazo[4,5-c]quinoline-2,4-diamine
   N2-butyl-1-(2-methylpropyl)-1H-imidazo[4,5-c]quinoline-2,4-diamine
   N2-butyl-N2-methyl-1-(2-methylpropyl)-1H-imidazo[4,5-c]quinoline-2,4-diamine
   N2-methyl-1-(2-methylpropyl)-N2-pentyl-1H-imidazo[4,5-c]quinoline-2,4-diamine
   N2-methyl-1-(2-methylpropyl)-N2-prop-2-enyl-1H-imidazo[4,5-c]quinoline-2,4-diamine
   1-(2-methylpropyl)-2-[(phenylmethyl)thio]-1H-imidazo[4,5-c]quinolin-4-amine
   1-(2-methylpropyl)-2-(propylthio)-1H-imidazo[4,5-c]quinolin-4-amine
   2-[[4-amino-1-(2-methylpropyl)-1H-imidazo[4,5-c]quinolin-2-yl](methyl)amino]ethanol
   2-[[4-amino-1-(2-methylpropyl)-1H-imidazo[4,5-c]quinolin-2-yl](methyl)amino]ethyl acetate
   4-amino-1-(2-methylpropyl)-1,3-dihydro-2H-imidazo[4,5-c]quinolin-2-one
   N2-butyl-1-(2-methylpropyl)-N4,N4-bis(phenylmethyl)-1H-imidazo[4,5-c]quinoline-2,4-diamine
   N2-butyl-N2-methyl-1-(2-methylpropyl)-N4,N4-bis(phenylmethyl)-1H-imidazo[4,5-c]quinoline-2,4-diamine
   N2 methyl-1-(2-methylpropyl)-N4,N4-bis(phenylmethyl)-1H-imidazo[4,5-c]quinoline-2,4-diamine
   N2,N2-dimethyl-1-(2-methylpropyl)-N4,N4-bis(phenylmethyl)-1H-imidazo[4,5-c]quinoline-2,4-diamine
   1-{4-amino-2-[methyl(propyl)amino]-1H-imidazo[4,5-c]quinolin-1-yl}-2-methylpropan-2-ol
   1-[4-amino-2-(propylamino)-1H-imidazo[4,5-c]quinolin-1-yl]-2-methylpropan-2-ol
   N4,N4-dibenzyl-1-(2-methoxy-2-methylpropyl)-N2-propyl-1H-imidazo[4,5-c]quinoline-2,4-diamine.
- Saponins [chapter 22 of ref. 136], which are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata (sarsaprilla), Gypsophilla paniculata* (brides veil), and *Saponaria ogicianalis* (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, (S17, QS18, QS21, QH-A, QH-B and QH-C. Preferably, the saponin is QS21. A method of production of QS21 is disclosed in ref. 108. Saponin formulations may also comprise a sterol, such as cholesterol [109]. Combinations of saponins and cholesterols can be used to form unique particles called immunostimulating complexs (ISCOMs) [chapter 23 of ref. 136]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 109-111. Optionally, the ISCOMS may be devoid of additional detergent [112]. A review of the development of saponin based adjuvants can be found in refs. 113 & 114. ISCOMs and free QS21 have both been reported to upregulate Th 1 responses.
- Bacterial ADP-ribosylating toxins (see above). The use of detoxified ADP-ribosylating toxins as parenteral adjuvants is described in reference 115. Some mutants have been reported to induce a polarized Th2-type response (*e.g*. LT-R72 in ref. 116; but cf. ref. 117), but others have been reported to induce mixed Th1/Th2-type responses (*e.g*. LT-K63) or Th1-type responses (*e.g*. LT-G192). The Th1/Th2 balance achieved by any particular mutant when given with an antigen by a chosen route and schedule can easily be assessed.
- Microparticles (*i.e*. a particle of ∼ 100nm to ∼150µm in diameter, more preferably -200nm to ∼30µm in diameter, and most preferably ∼500nm to ∼10µm in diameter) formed from materials that are biodegradable and non-toxic (*e.g*. a poly(a-hydroxy acid), a polyhydroxybutyric acid, a polyorthoester, a polyanhydride, a polycaprolactone, *etc.),* with poly(lactide-co-glycolide) being preferred, optionally treated to have a negatively-charged surface (*e.g*. with SDS) or a positively-charged surface (*e.g.* with a cationic detergent, such as CTAB). Encapsulation of antigens into PLG microparticles has been reported to favor a Th1-type response when compared to MF59 [118].
- Liposomes (Chapters 13 & 14 of ref. 136). Liposomes can elicit strong Th1 responses, particularly cationic liposomes containing mycobacterial lipids [119].
- A calcium salt, such as calcium phosphate (*e.g*. the "CAP" particles disclosed in ref. 120). Calcium salts have been reported to provide Th1 responses [121].
- Muramyl peptides, such as N-acetylmuramyl-L-threonyl-D-isoglutamine ("thr-MDP"), N-acetyl-normuramyl-L-alanyl-D-isoglutamine (nor-MDP), N-acetylglucsaminyl-N-acetylmuramyl-L-Al-D-isoglu-L-Ala-dipalmitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").
- An outer membrane protein proteosome preparation prepared from a first Gram-negative bacterium in combination with a liposaccharide (LPS) preparation derived from a second Gram-negative bacterium, wherein the outer membrane protein proteosome and LPS preparations form a stable non-covalent adjuvant complex. Such complexes include "IVX-908", a complex comprised of *Neisseria meningitidis* outer membrane and LPS.
- Methyl inosine 5'-monophosphate ("MIMP") [122].
- A polyhydroxlated pyrrolizidine compound [123], such as one having formula: 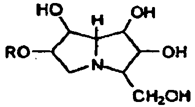 where R is selected from the group comprising hydrogen, straight or branched, unsubstituted or substituted, saturated or unsaturated acyl, alkyl (*e.g*. cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-*epi*-casuarine, 3,7-di*epi*-casuarine, *etc*. These compounds enhance Th 1 responses.
- A gamma inulin [124] or derivative thereof, such as algammulin. These adjuvants can promote both Th1 and Th2 immune responses [125].

A vitamin E compound. Vitamin E has a significant impact on the expression of genes involved in the Th1/Th2 balance, and vitamin E stimulation of immune cells can directly lead to increased IL-2 production (*i.e*. a Th1-type response) [126]. Natural vitamin E exists in eight different forms or isomers: four tocopherols and four tocotrienols. All isomers have a chromanol ring, with a hydroxyl group which can donate a hydrogen atom to reduce free radicals and a hydrophobic side chain which allows for penetration into biological membranes. There is an α, β, y and δ form of both the tocopherols and tocotrienols, determined by the number of methyl groups on the chromanol ring. Each form has its own biological activity, the measure of potency or functional use in the body. The tocopherols can take several forms *e.g.* different salts and/or isomers. Salts include organic salts, such as succinate, acetate, nicotinate, *etc.* D-α-tocopherol and DL-α-tocopherol can both be used. A preferred α-tocopherol is DL-α-tocopherol, and the preferred salt of this tocopherol is the succinate. As vitamin E compounds are usually oils, they can conveniently be included as a component in an oil-in-water emulsion, and oil-in-water emulsions containing tocopherols are reported to be Th1-inducing adjuvants.
- A compound of formula I, II or III, or a salt thereof: 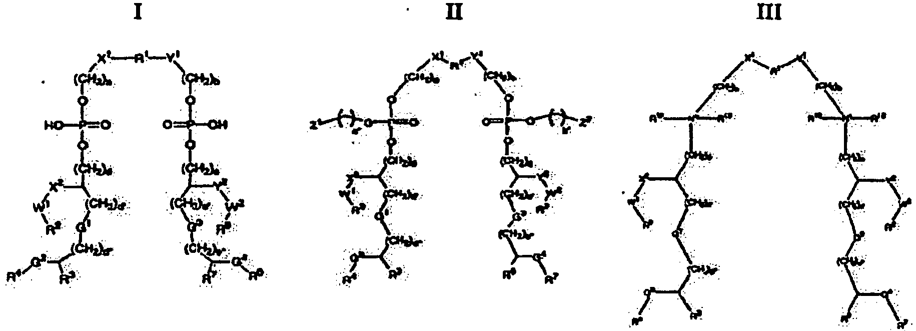 as defined in reference 127, such as 'ER 803058', 'ER 803732', 'ER 804053', ER 804058', 'ER 804059', 'ER 804442', 'ER 804680', 'ER 804764', ER 803022 or 'ER 804057' *e.g*.: 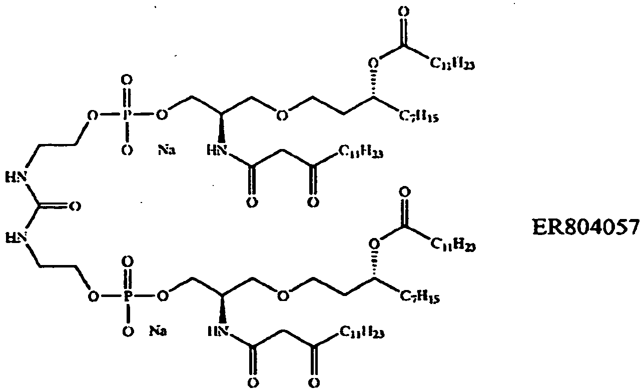 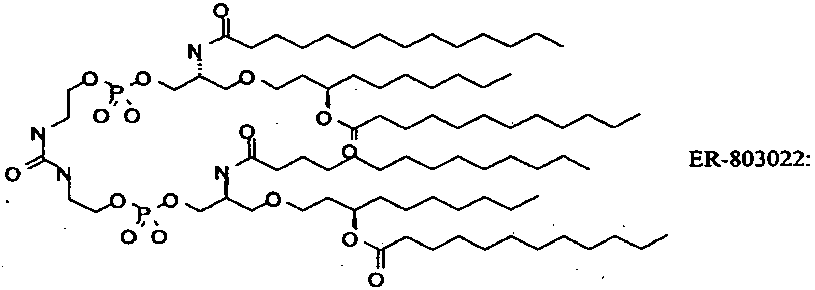
- Derivatives of lipid A from *Escherichia coli* such as OM-174 (described in refs. 128 & 129).
- A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [130].
- Compounds containing lipids linked to a phosphate-containing acyclic backbone, such as the TLR4 antagonist E5564 [131,132]:

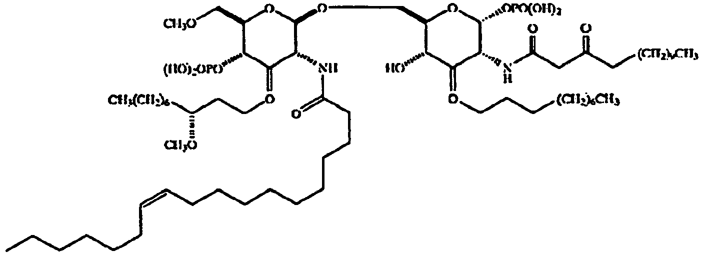

Compositions may include two or more of said adjuvants.

These and other adjuvant-active substances are discussed in more detail in references 136 & 137.

A preferred class of parenteral adjuvants is the group of oil-in-water emulsions, as described in more detail below. Within this group, squalene-containing emulsions are preferred, with the MF59 adjuvant being particularly suitable.

Thus a preferred parenteral immunization used with the invention involves multiple doses (*e.g*. 2) by intramuscular injection using a MF59 adjuvant.

Although the invention begins with mucosal administration, followed by parenteral administration, it does not exclude situations where mucosal administration is used again after parenteral administration. For example, a patient might receive three mucosal doses, two parenteral doses; and then another mucosal dose.

### Oil-in-water emulsion adjuvants

Oil-in-water emulsions are particularly useful as adjuvants. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidizer to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g.* obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100. Mixtures of surfactants can be used *e.g.* Tween 80/Span 85 mixtures.

Oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [133-135], as described in more detail in Chapter 10 ofref. 136 and chapter 12 of ref. 137. The MF59 emulsion advantageously includes citrate ions *e.g*. 10mM sodium citrate buffer.
- An emulsion of squalene, a tocopherol, and Tween 80. The emulsion may include phosphate buffered saline. It may also include Span 85 (*e.g.* at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets e.g. with an average diameter of between 100 and 250nm, preferably about 180nm.
- An emulsion of squalene, a tocopherol, and a Triton detergent (*e.g*. Triton X-100).
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [138] (0.05-1% Thr-MDP, 5% squalane, 2.5% Pluronic L121 and 0.2% polysorbate 80). It can also be used without the Thr-MDP, as in the "AF" adjuvant [139] (5% squalane, 1.25% Pluronic L121 and 0.2% polysorbate 80). Microfluidisation is preferred.
- An emulsion having from 0.5-50% of an oil, 0.1-10% of a phospholipid, and 0.05-5% of a non-ionic surfactant. As described in reference 140, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 141, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (*e.g*. QuilA or QS21) and a sterol (*e.g*. a cholesterol) are associated as helical micelles [142].

Squalene-containing emulsions are preferred, with the MF59 adjuvant being particularly suitable. Thus a preferred parenteral immunization used with the invention involves multiple doses (*e.g*. 2) by intramuscular injection using a MF59 adjuvant.

The emulsions may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g*. between 5:1 and 1:5) but is generally about 1:1.

### Aluminum salt adjuvants

The adjuvants known as aluminum hydroxide and aluminum phosphate may be used. These names are conventional, but are used for convenience only, as neither is a precise description of the actual chemical compound which is present (*e.g*. see chapter 9 of reference 136). The invention can use any of the "hydroxide" or "phosphate" adjuvants that are in general use as adjuvants.

The adjuvants known as "aluminum hydroxide" are typically aluminum oxyhydroxide salts, which are usually at least partially crystalline. Aluminum oxyhydroxide, which can be represented by the formula AIO(OH), can be distinguished from other aluminum compounds, such as aluminum hydroxide Al(OH)₃, by infrared (IR) spectroscopy, in particular by the presence of an adsorption band at 1070cm⁻¹ and a strong shoulder at 3090-3100cm⁻¹ [chapter 9 of ref. 136]. The degree of crystallinity of an aluminum hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g*. as seen in transmission electron micrographs) is typical for aluminum hydroxide adjuvants. The pI of aluminum hydroxide adjuvants is typically about 11 *i.e*. the adjuvant itself has a positive surface charge at physiological pH. Adsorptive capacities of between 1.8-2.6 mg protein per mg Al⁺⁺⁺ at pH 7.4 have been reported for aluminum hydroxide adjuvants.

The adjuvants known as "aluminum phosphate" are typically aluminum hydroxyphosphates, often also containing a small amount of sulfate (*i.e*. aluminum hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e.g*. when heated to 200°C) indicates the presence of structural hydroxyls [ch.9 of ref. 136].

The PO₄/Al³⁺ molar ratio of an aluminum phosphate adjuvant will generally be between 0.3 and 1.2, preferably between 0.8 and 1.2, and more preferably 0.95±0.1. The aluminum phosphate will generally be amorphous, particularly for hydroxyphosphate salts. A typical adjuvant is amorphous aluminum hydroxyphosphate with PO₄/Al molar ratio between 0.84 and 0.92, included at 0.6mg Al³⁺/ml. The aluminum phosphate will generally be particulate (*e.g*. plate-like morphology as seen in transmission electron micrographs). Typical diameters of the particles are in the range 0.5-20µm (*e.g*. about 5-10µm) after any antigen adsorption. Adsorptive capacities of between 0.7-1.5 mg protein per mg Al⁺⁺⁺ at pH 7.4 have been reported for aluminum phosphate adjuvants.

The point of zero charge (PZC) of aluminum phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminum phosphates used according to the invention will generally have a PZC of between 4.0 and 7.0, more preferably between 5.0 and 6.5 *e.g*. about 5.7.

Suspensions of aluminum salts used to prepare compositions of the invention may contain a buffer (*e.g*. a phosphate or a histidine or a Tris buffer), but this is not always necessary. The suspensions are, preferably sterile and pyrogen-free. A suspension may include free aqueous phosphate ions *e.g*. present at a concentration between 1.0 and 20 mM, preferably between 5 and 15 mM, and more preferably about 10 mM. The suspensions may also comprise sodium chloride.

The invention can use a mixture of both an aluminum hydroxide and an aluminum phosphate. In this case there may be more aluminium phosphate than hydroxide *e.g*. a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1mg/ml.

### Pharmaceutical composition

HIV-1 envelope antigens used with the invention will be administered as components in immunogenic compositions in order to elicit an immune response in a patient. The immune response can include a humoral (*e.g*. an antibody response, such as a neutralizing antibody response) and/or a cellular response. In a patient already infected with HIV, the immune response may reduce the severity of the infection (*e.g*. reduce viral load) and may even result in clearance of HIV infection (therapeutic immunization). In a patient who is not infected with HIV, the immune response may reduce the risk of future HIV infection and may even be protective against future HIV infection (prophylactic immunization). For therapeutic immunization, the effects arising from administration of the immunogenic composition may be augmented by, or also require, the use of other anti-HIV strategies *e.g*. the administration of antivirals, including but not limited to nucleoside reverse transcriptase inhibitors, non-nucleoside reverse transcriptase inhibitors, protease inhibitors, entry inhibitors, fusion inhibitors, *etc.*

Immunogenic compositions will include an immunologically effective amount of a HIV-1 envelope antigen. By 'immunologically effective amount', it is meant that the administration of that amount to an individual, either in a single dose or as part of a series, is effective for the desired treatment or prevention. This amount can vary depending upon the health and physical condition of the individual to be treated, age, the taxonomic group of individual to be treated (*e.g*. non-human primate, primate, *etc.),* the capacity of the individual's immune system to synthesize antibodies, the degree of protection desired, the formulation of the vaccine, the treating physician's assessment of the medical situation, and other relevant factors. It is expected that the amount will fall in a relatively broad range that can be determined through routine trials, and a typical quantity of antigen per dose is between 1µg and 1mg *e.g*. about 100µg per antigen per dose.

Immunogenic compositions disclosed herein are pharmaceutically acceptable. They usually include components in addition to the complexes *e.g*. they typically include one or more pharmaceutical carrier(s) and/or excipient(s). A thorough discussion of such components is available in ref. 143. Compositions will generally be in aqueous form.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions, particularly those for parenteral administration, will generally have an osmolality of between 200 mOsm/kg and 400 mOsm/kg, preferably between 240-360 mOsm/kg, and will more preferably fall within the range of 290-310 mOsm/kg.

Compositions may include one or more buffers. Typical buffers include: a phosphate buffer; a Tris buffer; a borate buffer; a succinate buffer; a histidine buffer; or a citrate buffer. Buffers will typically be included in the 5-20mM range.

The pH of a composition will generally be between 5 and 8, and more typically between 6 and 7.

The composition is preferably sterile. The composition is preferably non-pyrogenic *e.g.* containing <1 EU (endotoxin unit, a standard measure) per dose, and preferably <0.1 EU per dose. The composition is preferably gluten free.

Compositions disclosed herein may include detergent *e.g*. a polyoxyethylene sorbitan ester surfactant (known as 'Tweens'), an octoxynol (such as octoxynol-9 (Triton X-100) or t-octylphenoxypolyethoxyethanol), *etc.*

Vaccines for injection may be administered in a dosage volume of about 0.5ml.

Vaccines for intranasal administration may be administered in a dosage volume of about 0.3ml. This volume may be administered in a single nostril or be split between both nostrils.

### Patients

The invention is particularly suitable for use with human patients, but can also be used with other mammals for investigational purposes, for raising antisera, *etc.*

Typical patients will be adolescents (*e.g*. 12-17 years old) and adults (*e.g*. 18-55 years old).

### Kits of the disclosure

Where a composition includes an antigen and an adjuvant, these may be mixed during manufacture, or they may be mixed extemporaneously, at the time of delivery. In some cases, the adjuvant and antigen can be administered to a patient separately, without the need for mixing. Thus a combination of antigen and adjuvant may be provided in the form of a kit including the various components, either ready for mixing at the time of use, or ready for separate administration.

The components are physically separate from each other within the kit, and this separation can be achieved in various ways. For instance, the two components may be in two separate containers, such as vials. The contents of the two vials can then be mixed *e.g*. by removing the contents of one vial and adding them to the other vial, or by separately removing the contents of both vials and mixing them in a third container.

In a preferred arrangement, one of the kit components is in a syringe and the other is in a container such as a vial. The syringe can be used (*e.g*. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe *e.g.* a dual-chamber syringe, such as those disclosed in references 144-151 *etc.* When the syringe is actuated (*e.g*. during administration to a patient) then the contents of the two chambers are mixed. This arrangement avoids the need for a separate mixing step at the time of use.

The kit components will generally be in aqueous form. In some arrangements, a component (typically the antigen component rather than the adjuvant component) is in dry form (*e.g*. in a lyophilized form), with the other component being in aqueous form. The two components can be mixed in order to reactivate the dry component and give an aqueous composition for administration to a patient. A lyophilized component will typically be located within a vial rather than a syringe. Dried components may include stabilizers such as lactose, sucrose or mannitol, as well as mixtures thereof *e.g*. lactose/sucrose mixtures, sucrose/mannitol mixtures, *etc*. One possible arrangement uses an aqueous adjuvant component in a pre-filled syringe and a lyophilized antigen component in a vial.

### General

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x means, for example, x±10%.

Unless specifically stated, a process comprising a step of mixing two or more components does not require any specific order of mixing. Thus components can be mixed in any order. Where there are three components then two components can be combined with each other, and then the combination may be combined with the third component, *etc*.

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc*.), as well as other modifications known in the art. Polypeptides can occur as single chains or associated chains. Polypeptides can be naturally or non-naturally glycosylated (*i.e*. the polypeptide has a glycosylation pattern that differs from the glycosylation pattern found in the corresponding naturally occurring polypeptide).

Polypeptides for use with the invention can be prepared in many ways *e.g*. by chemical synthesis (in whole or in part), by digesting longer polypeptides using proteases, by translation from RNA, by purification from cell culture (*e.g*. from recombinant expression), from the organism itself (*e.g*. after bacterial culture, or direct from patients), *etc.* A preferred method for production of peptides <40 amino acids long involves *in vitro* chemical synthesis [152,153]. Solid-phase peptide synthesis is particularly preferred, such as methods based on tBoc or Fmoc [154] chemistry. Enzymatic synthesis [15] may also be used in part or in full. As an alternative to chemical synthesis, biological synthesis may be used *e.g.* the polypeptides may be produced by translation. This may be carried out *in vitro* or *in vivo.* Biological methods are in general restricted to the production of polypeptides based on L-amino acids, but manipulation of translation machinery (*e.g*. of aminoacyl tRNA molecules) can be used to allow the introduction of D-amino acids (or of other non natural amino acids, such as iodotyrosine or methylphenylalanine, azidohomoalanine, etc.) [156]. Where D-amino acids are included, however, it is preferred to use chemical synthesis. Polypeptides may have covalent modifications at the C-terminus and/or N-terminus.

Polypeptides can take various forms (*e.g*. native, fusions, glycosylated, non-glycosylated, lipidated, non-lipidated, phosphorylated, non-phosphorylated, myristoylated, non-myristoylated, monomeric, multimeric, particulate, denatured, *etc*.). For HIV-1 envelope antigen, oligomeric glycosylated polypeptides are preferred.

Polypeptides are preferably provided in purified or substantially purified form *i.e*. substantially free from other polypeptides (*e.g*. free from naturally-occurring polypeptides), particularly from other HIV or host cell polypeptides, and are generally at least about 50% pure (by weight), and usually at least about 90% pure *i.e*. less than about 50%, and more preferably less than about 10% (*e.g.* 5% or less) of a composition is made up of other expressed polypeptides.

### MODES FOR CARRYING OUT THE INVENTION

### Mouse experiments

Five groups of 10 Balb-c mice received the following:
- Groups 1-4 received 3 intranasal immunizations with doses of 0.5 µg, 5µg, 25 µg, or 50 µg of o-gp140ΔV2_{SF162} protein with 10 µg LTK63.
- Group 5 received 3 intranasal doses of 10 µg LTK63, as a control.

Intranasal doses were administered in a total aqueous volume of 40µl, at two week intervals (0, 2, & 4 weeks). All five groups then received two intramuscular boosters (6 & 8 weeks) with 25µg of o-gp140AV2_{SF162} protein in combination with a MF59 adjuvant (50 µl).

Serum anti-Env IgG and vaginal anti-Env IgA were measured after each immunization.

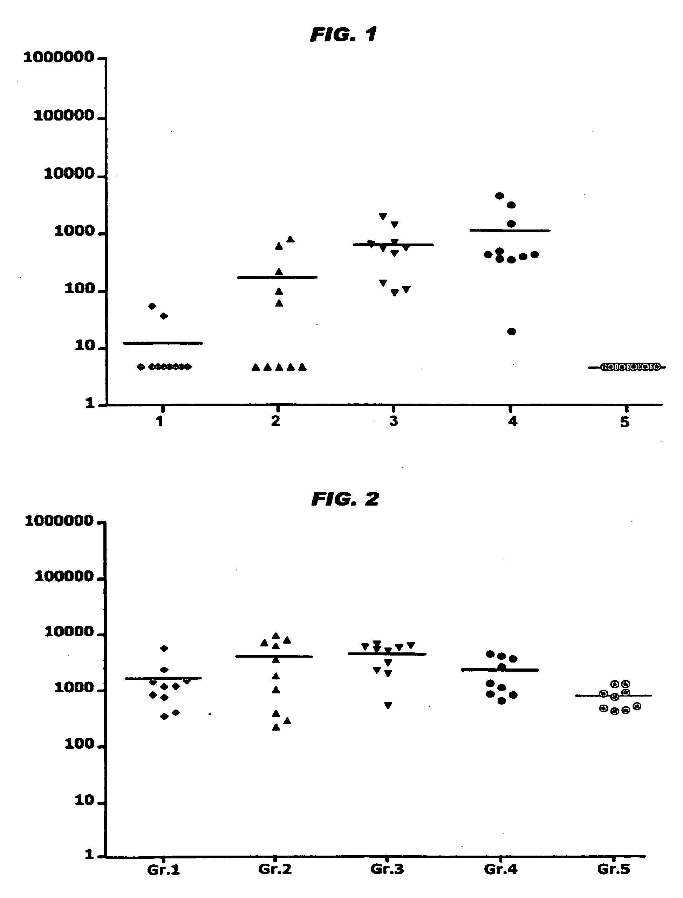

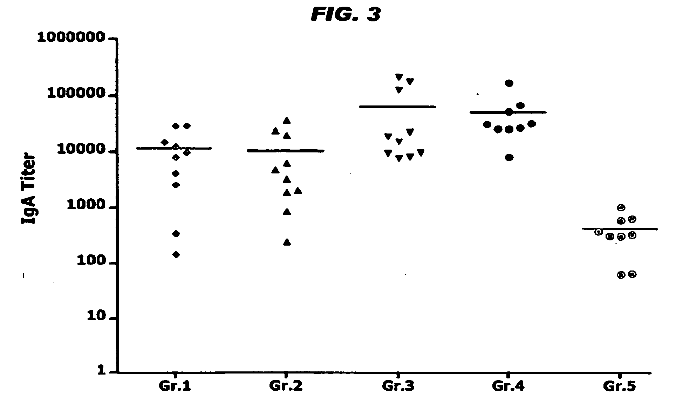

IgG levels 2 weeks after the third intranasal dose, immediately preceding the first intramuscular dose, are shown in Figure 1. IgG levels 2 weeks after the second intramuscular dose are in Figure 2. A comparison of Figures 1 and 2 shows that the intramuscular immunizations increase IgG titers in all groups. Good IgA responses were also seen after the fifth dose (Figure 3). For both IgG and IgA, the highest mean titer was seen in group 3 (25µg antigen).

### Human clinical trial

Three groups of 10 subjects (aged 18-45 years) receive (i) three nasal doses of LT-K63 (30µg per dose), (ii) o-gp140ΔV2_{SF162} protein (100µg per dose), or (iii) a mixture of both. All patients then receive two intramuscular doses of o-gp140ΔV2_{SF162} (100µg per dose) in a MF59 adjuvant.

For the intranasal doses, the separate components (antigen and/or adjuvant) are mixed before administration to a final volume of 284 µL, and this volume is equally distributed between left and right nostrils via a 200µl pipette.

For the intramuscular doses, a solution of o-gp140ΔV2_{SF162} is diluted to give 100µg at 2x concentration. This solution is then mixed at a 1:1 volume ratio with MF59, to give a final solution.

The five doses are administered at time 0, 4 weeks, 8 weeks, 16 weeks and 28 weeks.

To assess immunogenicity, blood samples are taken before time 0, at time 0, week 4 minus 1 day, week 8 minus 1 day, then at weeks 10, 12, 16, 28 & 32. Nasal and vaginal (for females) samples are taken at the same times.

Total serum IgG antibody responses against HIV-1 Env are measured in the blood samples using standard indirect ELISA. Similarly, total nasal and vaginal secretory IgA antibody responses against HIV-1 Env are measured using standard indirect ELISA. Moreover, T-cell responses to HIV are measured by assessing interferon-γ release by ELISpot assay of PBMCs in response to stimulation with HIV peptides. Similarly, serum functional antibody responses are measured by assessing virus neutralisation against the homologous HIV-1 strain.

### REFERENCES

[1] WO 03/059385.
[2] WO 02/080648.
[3] Robertson et al. (2000) Science 288:55-56.
[4] *http:*//*hiv-web.lanl.gov*/
[5] WO 00/39302.
[6] WO 03/020876.
[7] WO 2005/007808.
[8] WO 03/004620.
[9] WO 00/39304.
[10] U.S. Patent No. 5,792,459.
[11] Zhang et al. (2001) J. Biol. Chem. 276:39577-39585.
[12] Earl et al. (2001) J. Virol. 75:645-653.
[13] Barnett et al. (2001) J. Virol. 75:5526-5540.
[14] Srivastava et al. (2003) J. Virol. 77:2310-2320.
[15] WO 9/15238.
[16] Geysen et al. (1984) Proc. Natl. Acad. Sci. USA 81:3998-4002.
[17] Carter (1994) Methods Mol. Biol. 36:207-223.
[18] Jameson, BA et al. 1988, CABIOS 4(1):181-186.
[19] Raddrizzani & Hammer (2000) Brief Bioinform. 1(2):179-189.
[20] De Lalla et al. (1999) J. Immunol. 163:1725-1729.
[21] Brusic et al. (1998) Bioinformatics 14(2):121-130
[22] Meister et al. (1995) Vaccine 13(6):581-591.
[23] Roberts et al. (1996) AIDS Res. Hum. Retroviruses 12(7):593-610.
[24] Maksyutov & Zagrebelnaya (1993) Comput Appl. Biosci. 9(3):291-297.
[25] Feller & de la Cruz (1991) Nature 349(6311):720-721.
[26] Hopp (1993) Peptide Research 6:183-190.
[27] Welling et al. (1985) FEBS Lett. 188:215-218.
[28] Davenport et al. (1995) Immunogenetics 42:392-397.
[29] Needleman& Wunsch (1970) J. Mol. Biol. 48:443-453.
[30] Rice et al. (2000) Trends Genet. 16:276-277.
[31] Ensoli et al. (2005) Microbes Infect. 7:1392-1399.
[32] WO 2005/090391.
[33] Greenbaum et al. (2004) Vaccine 22:2566-2577.
[34] Zurbriggen et al. (2003) Expert Rev. Vaccines 2:295-304.
[35] Piascik (2003) J. Am. Pharm. Assoc. (Wash DC). 43:728-730.
[36] Mann et al. (2004) Vaccine 22:2425-2429.
[37] Almeida & Alpar (1996) J. Drug Targeting 3:455-467.
[38] Agarwal & Mishra (1999) Indian J. Exp. Biol. 37:6-16.
[39] Michetti (1998) J. Gastroenterol. [Suppl X]:66-68.
[40] Pizza et al. (2000) Int. J. Med. Microbiol. 290:455-461.
[41] WO 95/17211.
[42] WO 99/52549.
[43] WO 01/21207.
[44] WO 01/21152.
[45] WO 00/50078.
[46] Singh et al. (2001) J. Cont. Release 70:267-276.
[47] WO 99/27960.
[48] WO 99/27960.
[49] WO00/62800.
[50] U.S. Patent No. 6,090,406
[51] US 5,916,588.
[52] EP-A-0626169.
[53] Johnson et al. (1999) Bioorg. Med. Chem. Lett. 9:2273-2278.
[54] Evans et al. (2003) Expert Rev. Vaccines 2:219-229.
[55] Andrianov et al. (1998) Biomaterials 19:109-115.
[56] Payne et al. (1998) Adv. Drug Delivery Review 31:185-196.
[57] Burdman et al. (eds.) (1995) Vaccine Design: The Subunit and Adjuvant Approach, 1st edition (Springer). ISBN: 030644867X
[58] WO 98/18928.
[59] Del Giudice et al. (1998) Molecular Aspects of Medicine, vol. 19, number 1.
[60] Park et al. (2000) Exp. Mol. Med. 32:72-78.
[61] Domenighini et al. (1995) Mol. Microbiol. 15:1165-1167.
[62] WO 01/22972.
[63] Kandimalla et al. (2003) Nucleic Acids Research 31:2393-2400.
[64] WO 02/26757.
[65] WO 99/62923.
[66] Krieg (2003) Nature Medicine 9:831-835.
[67] McCluskie et al. (2002) FEMS Immunol. Med. Microbiol. 32:179-185.
[68] WO 98/40100.
[69] U.S. Patent No. 6,207,646.
[70] U.S. Patent No. 6,239,116.
[71] U.S. Patent No. 6,429,199.
[72] Kandimalla et al. (2003) Biochem. Soc. Trans. 31 (part 3):654-658.
[73] Blackwell et al. (2003) J. Immunol. 170:4061-4068.
[74] Krieg (2002) Trends Immunol. 23:64-65.
[75] WO 01/95935.
[76] Kandimalla et al. (2003) BBRC 306:948-953.
[77] Bhagat et al. (2003) BBRC 300:853-861.
[78] WO 03/035836.
[79] Myers et al. (1990) pages 145-156 of Cellular and molecular aspects of endotoxin reactions*.*
[80] Ulrich (2000) Chapter 16 (pages 273-282) of reference 137.
[81] Johnson et al. (1999) J. Med. Chem. 42:4640-4649.
[82] Baldrick et al. (2002) Regulatory Toxicol. Pharmacol. 35:398-413.
[83] UK patent application GB-A-2220211.
[84] Wheeler et al. (2001) International Archives of Allergy and Immunology 126: 35-139
[85] U.S. Patent No. 4,680,338.
[86] U.S. Patent No. 4,988,815.
[87] WO 92/15582.
[88] Stanley (2002) Clin. Exp. Dermatol. 27:571-577.
[89] Wu et al. (2004) Antiviral Res. 64(2):79-83.
[90] Vasilakos et al. (2000) Cell. Immunol.. 204(1):64-74.
[91] U.S. Patent Nos. 4,689,338; 4,929,624; 5,238,944; 5,266,575; 5,268,376; 5,346,905; 5,352,784; 5,389,640; 5,395,937; 5,482,936; 5,494,916; 5,525,612; 6,083,505; 6,440,992; 6,627,640; 6,656,938; 6,660,735; 6,660,747; 6,664,260; 6,664,264; 6,664,265; 6,667,312; 6,670,372; 6,677,347; 6,677,348; 6,677,349; 6,683,088; 6,703,402; 6,743,920; 6,800,624; 6,809,203; 6,888,000; and 6,924,293.
[92] Jones (2003) Curr. Opin. Investig. Drugs 4:214-218.
[93] WO 2004/060308.
[94] WO 2004/064759.
[95] U.S. Patent No. 6,355,271.
[96] WO 00/23105.
[97] U.S. Patent No. 6,924,271.
[98] U.S. Publication No. 2005/0070556.
[99] U.S. Patent No. 5,658,731.
[100] U.S. Patent No. 5,011,828.
[101] WO 2004/87153.
[102] U.S. Patent No. 6,605,617.
[103] WO 02/18383.
[104] WO 2004/018455.
[105] WO 03/082272.
[106] WO 2006/002422.
[107] Thompson et al. (2005) Journal of Leukocyte Biology 78:1273-1280
[108] U.S. Patent No. 5,057,540.
[109] WO 96/33739.
[110] EP-A-0109942.
[111] WO 96/11711.
[112] WO 00/07621.
[113] Barr et al. (1998) Advanced Drug Delivery Reviews 32:247-271.
[114] Sjolanderet et al. (1998) Advanced Drug Delivery Reviews 32:321-338.
[115] WO 98/42375.
[116] Feng et al. (2005) Acta Biochim Biophys Sin (Shanghai). 37(2):126-32.
[117] Barackman et al. (1999) Infect Immun 67(8):4276-9.
[118] Singh et al. (1998) Vaccine 16(19):1822-7.
[119] Rosenkrands et al. (2005) Infect Immun 73(9):5817-26.
[120] US patent 6355271.
[121] He et al. (2000) Clin Diagn Lab Immunol 7(6): 899-903.
[122] Signorelli & Hadden (2003) Int. Immunopharmacol. 3(8):1177-1186.
[123] WO 2004/064715.
[124] Cooper (1995) Pharm. Biotechnol. 6:559-5 80.
[125] Silva et al. (2004) Immunol Cell Biol 82(6):611-6.
[126] Han et al. (2004) Ann N YAcad Sci 1031:96-101.
[127] WO 03/011223.
[128] Meraldi et al. (2003) Vaccine 21:2485-2491.
[129] Pajak et al. (2003) Vaccine 21:836-842.
[130] U.S. Patent No. 6,586,409.
[131] Wong et al. (2003) J. Clin. Pharmacol. 43(7):735-742.
[132] U.S. Publication No. 2005/0215517.
[133] WO 90/14837.
[134] Podda & Del Giudice (2003) Expert Rev. Vaccines 2:197-203.
[135] Podda (2001) Vaccine 19: 2673-2680.
[136]Burdman et al. (eds.) (1995) Vaccine Design: The Subunit and Adjuvant Approach, 1st edition (Springer). ISBN: 030644867X.
[137]O'Hagan (ed.) (2000) Vaccine Adjuvants: Preparation Methods and Research Protocols (Volume 42 of Methods in Molecular Medicine series) (Humana Press). ISBN: 0896037355.
[138] Allison & Byars (1992) Res. Immunol. 143:519-525.
[139] Hariharan et al. (1995) Cancer Res. 55:3486-3489.
[140] WO 95/11700.
[141] U.S. Patent No. 6,080,725.
[142] WO 2005/097181.
[143] Gennaro (2000) Remington: The Science and Practice of Pharmacy, 20th edition (Lippincott Williams & Wilkens). ISBN: 0683306472.
[144] WO 2005/089837.
[145] U.S. Patent No. 6,692,468.
[146] WO 00/07647.
[147] WO 99/17820.
[148] U.S. Patent No. 5,971,953.
[149] U.S. Patent No. 4,060,082.
[150] EP-A-0520618.
[151] WO 98/01174.
[152]Bodanszky (1993) Principles of Peptide Synthesis, 2nd edition (Springer-Verlag). ISBN: 0387564314.
[1 53]Fields et al. (1997) Methods In Enzymology, Volume 289: Solid-Phase Peptide Synthesis, 1st edition (Academic Press). ISBN: 0121821900.
[154]Chan & White (2000) Fmoc Solid Phase Peptide Synthesis (Oxford University Press). ISBN: 0199637245.
[155] Kullmann (1987) Enzymatic Peptide Synthesis. ISBN: 0849368413.
[156] Ibba(1996) Biotechnol. Genet. Eng. Rev. 13:197-216.
[157] Vajdy et al. (2004) Aids research and human retroviruses 20(11): 1269-1281

## Claims

1. An HIV-1 envelope protein (Env) antigen, for use in a therapeutic or prophylactic method for raising serum and mucosal antibody responses in a patient, **characterised in that** the method comprises: (i) administering a series of priming doses of the HIV-1 envelope protein (Env) antigen to the patient via a mucosal route, wherein the series consists of three doses; and then (ii) administering two boosting doses of an HIV-1 envelope protein (Env) antigen to the patient via the intramuscular route, wherein each immunization uses the same HIV-1 envelope protein (Env) antigen, wherein the first intramuscular dose is administered at least 2 weeks after the final dose in the mucosal series, and wherein administering said boosting doses increases both the serum and mucosal antibody responses induced by said priming doses.

2. The use of an HIV-1 envelope protein (Env) antigen in the manufacture of a medicament for raising serum and mucosal antibody responses in a patient by a method comprising: (i) administering a series of priming doses of the HIV-1 envelope protein (Env) antigen to the patient via a mucosal route, wherein the series consists of three doses; and then (ii) administering two boosting doses of an HIV-1 envelope protein (Env) antigen to the patient via the intramuscular route, wherein each immunization uses the same HIV-1 envelope protein (Env) antigen, wherein the first intramuscular dose is administered at least 2 weeks after the final dose in the mucosal series, and wherein administering said boosting doses increases both the serum and mucosal antibody responses induced by said priming doses.

3. An HIV-1 envelope protein (Env) antigen, for use in a therapeutic or prophylactic method for raising serum and mucosal antibody responses in a patient, **characterised in that** the method comprises: administering two boosting doses of the HIV-1 envelope protein (Env) antigen to the patient via the intramuscular route, wherein the patient has previously received a series of priming doses of an HIV-1 envelope protein (Env) antigen via a mucosal route, wherein the mucosal series consists of three doses, wherein each immunization uses the same HIV-1 envelope protein (Env) antigen, wherein the first intramuscular dose is administered at least 2 weeks after the final dose in the mucosal series, and wherein administering said boosting doses increases both the serum and mucosal antibody responses induced by said priming doses.

4. The use of an HIV-1 envelope protein (Env) antigen in the manufacture of a medicament for raising serum and mucosal antibody responses in a patient who has previously received a series of priming doses of an HIV-1 envelope protein (Env) antigen via a mucosal route, wherein the mucosal series consists of three doses, wherein the medicament is for administration to the patient via the intramuscular route, wherein two intramuscular boosting doses are administered to said patient, wherein the first intramuscular dose is administered at least 2 weeks after the final dose in the mucosal series, wherein each immunization uses the same HIV-1 envelope protein (Env) antigen, and wherein administering said boosting doses increases both the serum and mucosal antibody responses induced by said priming doses.

5. The antigen according to claim 1 or 3 for the use according to claim 1 or 3, or the use of claim 2 or 4, wherein the HIV-1 envelope protein (Env) antigens are adjuvanted.

6. The antigen according to any of claims 1, 3 and 5 for the use according to any of claims 1, 3 and 5, or the use of any of claims 2, 4, and 5, wherein mucosal immunizations include a detoxified mutant of *E.coli* heat labile toxin as adjuvant.

7. The antigen according to any of claims 1, 3, 5 and 6 for the use according to any of claims 1, 3, 5 and 6, or the use of any of claims 2 and 4-6, wherein parenteral immunizations include an oil-in-water emulsion as adjuvant.

8. The antigen according to any of claims 1, 3 and 5-7 for the use according to any of claims 1, 3 and 5-7, or the use of any of claims 2 and 4-7, wherein the mucosal immunization has three intranasal doses of a composition with between 10µg and 50µg of LT-K63 adjuvant per dose.

9. The antigen according to any of claims 1, 3 and 5-8 for the use according to any of claims 1, 3, and 5-8, or the use of any of claims 2 and 4-8, wherein the parenteral immunization has two intramuscular doses of a composition with a squalene-in-water emulsion.

10. The antigen according to any of claims 1, 3 and 5-9 for the use according to any of claims 1, 3 and 5-9, or the use of any of claims 2 and 4-9, wherein the patient is (a) a human aged between 12 and 55 years old; (b) not infected with HIV-1; or (c) infected with HIV-1.

11. The antigen according to any of claims 1, 3 and 5-9 for the use according to any of claims 1, 3 and 5-10, or the use of any of claims 2 and 4-10, wherein the envelope protein (Env) antigen is o=gp140ΔV2_{SF162}.

12. The antigen according to any of claims 1, 3 and 5-9 for the use according to any of claims 1, 3 and 5-11, or the use of any of claims 2 and 4-11, wherein mucosal immunizations are via the intranasal route.

## Patentansprüche

1. HIV-1-Hüllprotein(Env)-Antigen zur Verwendung in einem therapeutischen oder prophylaktischen Verfahren zur Erzeugung von Serum- und mukosalen Antikörperantworten bei einem Patienten, **dadurch gekennzeichnet, dass** man bei dem Verfahren (i) dem Patienten eine Reihe von Priming-Dosen des HIV-1-Hüllprotein(Env)-Antigens über einen mukosalen Weg verabreicht, wobei die Reihe aus drei Dosen besteht, und danach (ii) dem Patienten zwei Boosting-Dosen eines HIV-1-Hüllprotein(Env)-Antigens über den intramuskulären Weg verabreicht, wobei bei den Immunisierungen jeweils das gleiche HIV-1-Hüllprotein(Env)-Antigen verwendet wird, wobei die erste intramuskuläre Dosis mindestens 2 Wochen nach der letzten Dosis in der mukosalen Reihe verabreicht wird und wobei durch Verabreichen der Boosting-Dosen sowohl die Serumals auch die mukosalen Antikörperantworten, die durch die Priming-Dosen induziert wurden, gesteigert werden.

2. Verwendung eines HIV-1-Hüllprotein(Env)-Antigens bei der Herstellung eines Arzneimittels zur Erzeugung von Serum- und mukosalen Antikörperantworten bei einem Patienten mit einem Verfahren, bei dem man (i) dem Patienten eine Reihe von Priming-Dosen des HIV-1-Hüllprotein(Env)-Antigens über einen mukosalen Weg verabreicht, wobei die Reihe aus drei Dosen besteht, und danach (ii) dem Patienten zwei Boosting-Dosen eines HIV-1-Hüllprotein(Env)-Antigens über den intramuskulären Weg verabreicht, wobei bei den Immunisierungen jeweils das gleiche HIV-1-Hüllprotein(Env)-Antigen verwendet wird, wobei die erste intramuskuläre Dosis mindestens 2 Wochen nach der letzten Dosis in der mukosalen Reihe verabreicht wird und wobei durch Verabreichen der Boosting-Dosen sowohl die Serum- als auch die mukosalen Antikörperantworten, die durch die Priming-Dosen induziert wurden, gesteigert werden.

3. HIV-1-Hüllprotein(Env)-Antigen zur Verwendung in einem therapeutischen oder prophylaktischen Verfahren zur Erzeugung von Serum- und mukosalen Antikörperantworten bei einem Patienten, **dadurch gekennzeichnet, dass** man bei dem Verfahren dem Patienten zwei Boosting-Dosen des HIV-1-Hüllprotein(Env)-Antigens über den intramuskulären Weg verabreicht, wobei der Patient zuvor eine Reihe von Priming-Dosen eines HIV-1-Hüllprotein(Env)-Antigens über einen mukosalen Weg erhalten hat, wobei die mukosale Reihe aus drei Dosen besteht, wobei bei den Immunisierungen jeweils das gleiche HIV-1-Hüllprotein(Env)-Antigen verwendet wird, wobei die erste intramuskuläre Dosis mindestens 2 Wochen nach der letzten Dosis in der mukosalen Reihe verabreicht wird und wobei durch Verabreichen der Boosting-Dosen sowohl die Serum- als auch die mukosalen Antikörperantworten, die durch die Priming-Dosen induziert wurden, gesteigert werden.

4. Verwendung eines HIV-1-Hüllprotein(Env)-Antigens bei der Herstellung eines Arzneimittels zur Erzeugung von Serum- und mukosalen Antikörperantworten bei einem Patienten, der zuvor eine Reihe von Priming-Dosen eines HIV-1-Hüll-protein(Env)-Antigens über einen mukosalen Weg erhalten hat, wobei die mukosale Reihe aus drei Dosen besteht, wobei das Arzneimittel für die Verabreichung an den Patienten über den intramuskulären Weg vorgesehen ist, wobei dem Patienten zwei intramuskuläre Boosting-Dosen verabreicht werden, wobei die erste intramuskuläre Dosis mindestens 2 Wochen nach der letzten Dosis in der mukosalen Reihe verabreicht wird, wobei bei den Immunisierungen jeweils das gleiche HIV-1-Hüllprotein(Env)-Antigen verwendet wird und wobei durch Verabreichen der Boosting-Dosen sowohl die Serum- als auch die mukosalen Antikörperantworten, die durch die Priming-Dosen induziert wurden, gesteigert werden.

5. Antigen nach Anspruch 1 oder 3 zur Verwendung nach Anspruch 1 oder 3 oder Verwendung gemäß Anspruch 2 oder 4, wobei die HIV-1-Hüllprotein(Env)-Antigene mit einem Adjuvans versehen sind.

6. Antigen nach einem der Ansprüche 1, 3 und 5 zur Verwendung nach einem der Ansprüche 1, 3 und 5 oder Verwendung gemäß einem der Ansprüche 2, 4 und 5, wobei mukosale Immunisierungen eine detoxifizierte Mutante von hitzelabilem Toxin aus E.coli als Adjuvans umfassen.

7. Antigen nach einem der Ansprüche 1, 3, 5 und 6 zur Verwendung nach einem der Ansprüche 1, 3, 5 und 6 oder Verwendung gemäß einem der Ansprüche 2 und 4-6, wobei parenterale Immunisierungen eine Öl-in-Wasser-Emulsion als Adjuvans umfassen.

8. Antigen nach einem der Ansprüche 1, 3 und 5-7 zur Verwendung nach einem der Ansprüche 1, 3 und 5-7 oder Verwendung gemäß einem der Ansprüche 2 und 4-7, wobei die mukosale Immunisierung drei intranasale Dosen einer Zusammensetzung mit zwischen 10 µg und 50 µg LT-K63-Adjuvans pro Dosis aufweist.

9. Antigen nach einem der Ansprüche 1, 3 und 5-8 zur Verwendung nach einem der Ansprüche 1, 3 und 5-8 oder Verwendung gemäß einem der Ansprüche 2 und 4-8, wobei die parenterale Immunisierung zwei intramuskuläre Dosen einer Zusammensetzung mit einer Squalen-in-Wasser-Emulsion aufweist.

10. Antigen nach einem der Ansprüche 1, 3 und 5-9 zur Verwendung nach einem der Ansprüche 1, 3 und 5-9 oder Verwendung gemäß einem der Ansprüche 2 und 4-9, wobei der Patient (a) ein Mensch im Alter zwischen 12 und 55 Jahren ist; (b) nicht mit HIV-1 infiziert ist; oder (c) mit HIV-1 infiziert ist.

11. Antigen nach einem der Ansprüche 1, 3 und 5-10 zur Verwendung nach einem der Ansprüche 1, 3 und 5-10 oder Verwendung gemäß einem der Ansprüche 2 und 4-10, wobei es sich bei dem Hüllprotein(Env)-Antigen um o-gp140ΔV2_{SF162} handelt.

12. Antigen nach einem der Ansprüche 1, 3 und 5-11 zur Verwendung nach einem der Ansprüche 1, 3 und 5-11 oder Verwendung gemäß einem der Ansprüche 2 und 4-11, wobei mukosale Immunisierungen über den intranasalen Weg erfolgen.

## Revendications

1. Antigène protéine d'enveloppe (Env) du VIH-1, pour utilisation dans une méthode thérapeutique ou prophylactique destinée à augmenter les réponses anticorps sérique et muqueuse chez un patient, **caractérisé en ce que** la méthode comprend : (i) l'administration d'une série de doses d'amorçage de l'antigène protéine d'enveloppe (Env) du VIH-1 au patient par voie muqueuse, la série consistant en trois doses ; puis (ii) l'administration de deux doses de rappel d'un antigène protéine d'enveloppe (Env) du VIH-1 au patient par voie intramusculaire, chaque immunisation utilisant le même antigène protéine d'enveloppe (Env) du VIH-1, la première dose intramusculaire étant administrée au moins 2 semaines après la dose finale de la série muqueuse, et l'administration desdites doses de rappel augmentant la réponse anticorps, tant sérique que muqueuse, induite par lesdites doses d'amorçage.

2. Utilisation d'un antigène protéine d'enveloppe (Env) du VIH-1 dans la fabrication d'un médicament destiné à augmenter les réponses anticorps sérique et muqueuse chez un patient, par une méthode comprenant : (i) l'administration d'une série de doses d'amorçage de l'antigène protéine d'enveloppe (Env) du VIH-1 au patient par voie muqueuse, la série consistant en trois doses ; puis (ii) l'administration de deux doses de rappel d'un antigène protéine d'enveloppe (Env) du VIH-1 au patient par voie intramusculaire, chaque immunisation utilisant le même antigène protéine d'enveloppe (Env) du VIH-1, la première dose intramusculaire étant administrée au moins 2 semaines après la dose finale de la série muqueuse, et l'administration desdites doses de rappel augmentant la réponse anticorps, tant sérique que muqueuse, induite par lesdites doses d'amorçage.

3. Antigène protéine d'enveloppe (Env) du VIH-1, pour utilisation dans une méthode thérapeutique ou prophylactique pour augmenter les réponses anticorps sérique et muqueuse chez un patient, **caractérisé en ce que** la méthode comprend : l'administration de deux doses de rappel de l'antigène protéine d'enveloppe (Env) du VIH-1 au patient par voie intramusculaire, le patient ayant au préalable reçu une série de doses d'amorçage d'un antigène protéine d'enveloppe (Env) du VIH-1 par voie muqueuse, la série muqueuse consistant en trois doses, chaque immunisation utilisant le même antigène protéine d'enveloppe (Env) du VIH-1, la première dose intramusculaire étant administrée au moins 2 semaines après la dose finale de la série muqueuse, et l'administration desdites doses de rappel augmentant la réponse anticorps, tant sérique que muqueuse, induite par lesdites doses d'amorçage.

4. Utilisation d'un antigène protéine d'enveloppe (Env) du VIH-1 dans la fabrication d'un médicament pour augmenter les réponses anticorps sérique et muqueuse chez un patient qui a reçu au préalable une série de doses d'amorçage d'un antigène protéine d'enveloppe (Env) du VIH-1 par voie muqueuse, la série muqueuse consistant en trois doses, le médicament étant destiné à une administration au patient par voie intramusculaire, deux doses de rappel intramusculaires étant administrées audit patient, la première dose intramusculaire étant administrée au moins 2 semaines après la dose finale de la série muqueuse, chaque immunisation utilisant le même antigène protéine d'enveloppe (Env) du VIH-1, et l'administration desdites doses de rappel augmentant la réponse anticorps, tant sérique que muqueuse, induite par lesdites doses d'amorçage.

5. Antigène selon la revendication 1 ou 3 pour l'utilisation selon la revendication 1 ou 3, ou utilisation de la revendication 2 ou 4, les antigènes protéines d'enveloppe (Env) du VIH-1 étant adjuvantés.

6. Antigène selon l'une quelconque des revendications 1, 3 et 5 pour l'utilisation selon l'une quelconque des revendications 1, 3 et 5, ou utilisation de l'une quelconque des revendications 2, 4 et 5, les immunisations muqueuses comprenant en tant qu'adjuvant un mutant détoxiqué d'une toxine thermolabile d'E. coli.

7. Antigène selon l'une quelconque des revendications 1, 3, 5 et 6 pour l'utilisation selon l'une quelconque des revendications 1, 3, 5 et 6, ou utilisation de l'une quelconque des revendications 2 et 4-6, les immunisations parentérales comprenant en tant qu'adjuvant une émulsion de type huile-dans-l'eau.

8. Antigène selon l'une quelconque des revendications 1, 3 et 5-7 pour l'utilisation selon l'une quelconque des revendications 1, 3 et 5-7, ou utilisation de l'une quelconque des revendications 2 et 4-7, l'immunisation muqueuse comportant trois doses intranasales d'une composition contenant, par dose, de 10 à 50 µg de l'adjuvant LT-K63.

9. Antigène selon l'une quelconque des revendications 1, 3 et 5-8 pour l'utilisation selon l'une quelconque des revendications 1, 3 et 5-8, ou utilisation de l'une quelconque des revendications 2 et 4-8, l'immunisation parentérale comportant deux doses intramusculaires d'une composition contenant une émulsion squalène-dans-l'eau.

10. Antigène selon l'une quelconque des revendications 1, 3 et 5-9 pour l'utilisation selon l'une quelconque des revendications 1, 3 et 5-9, ou utilisation de l'une quelconque des revendications 2 et 4-9, le patient (a) étant un humain âgé de 12 à 55 ans ; (b) n'étant pas infecté par le VIH-1 ; ou (c) étant infecté par le VIH-1.

11. Antigène selon l'une quelconque des revendications 1, 3 et 5-10 pour l'utilisation selon l'une quelconque des revendications 1, 3 et 5-10, ou utilisation de l'une quelconque des revendications 2 et 4-10, l'antigène protéine d'enveloppe (Env) étant l'o-gp140ΔV2_{SF162}.

12. Antigène selon l'une quelconque des revendications 1, 3 et 5-11 pour l'utilisation selon l'une quelconque des revendications 1, 3 et 5-11, ou utilisation de l'une quelconque des revendications 2 et 4-11, les immunisations muqueuses s'effectuant par voie intranasale.
